# EUROPÄISCHE PATENTSCHRIFT

(11) **EP 2 134 170 B1**
(45) Veröffentlichungstag und Bekanntmachung des Hinweises auf die Patenterteilung: **03.06.2015**
(21) Anmeldenummer: 08716375.4
(22) Anmeldetag: 08.03.2008
(51) Int. Cl.: A01N 37/52, C07C 205/38, C07C 217/90, A01N 43/78, A01N 43/82, C07C 257/12

(54) **FLUORALKYLPHENYLAMIDINE UND DEREN VERWENDUNG ALS FUNGIZIDE**
FLUORALKYLPHENYLAMIDINES AND THEIR USE AS FUNGICIDES
FLUOROALKYLPHENYLAMIDINES ET LEUR UTILISATION COMME FONGICIDES

(30) Priorität: 12.03.2007 EP 07005002
(43) Veröffentlichungstag der Anmeldung: 23.12.2009
(73) Patentinhaber: Bayer Intellectual Property GmbH, 40789 Monheim am Rhein (DE)
(72) Erfinder: KUNZ, Klaus, 40625 Düsseldorf (DE); GREUL, Jörg, Nico, 42799 Leichlingen (DE); HEINEMANN, Ulrich, 42799 Leichlingen (DE); MANSFIELD, Darren, James, 51515 Kürten (DE); MATTES, Amos, 40674 Langenfeld (DE); ORT, Oswald, 51375 Leverkusen (DE); SEITZ, Thomas, 40764 Langenfeld (DE); MORADI, Wahed, Ahmed, 40789 Monheim (DE); WACHENDORFF-NEUMANN, Ulrike, 56566 Neuwied (DE); DAHMEN, Peter, 41470 Neuss (DE); VOERSTE, Arnd, 50674 Köln (DE)
(74) Vertreter: BIP Patents
(86) Internationale Anmeldenummer: PCT/EP2008/001863
(87) Internationale Veröffentlichungsnummer: WO 2008/110314

(56) Entgegenhaltungen:
- WO-A-00/46184
- WO-A-2005/094582
- WO-A-2007/031507
- WO-A-2007/031508
- WO-A-2007/031513
- WO-A-2007/031524

## Beschreibung

Die vorliegende Erfindung betrifft Fluoralkylphenylamidine der allgemeinen Formel (I), ein Verfahren zu deren Herstellung, die Verwendung der erfindungsgemäßen Amidine zum Bekämpfen von unerwünschten Mikroorganismen, sowie ein Mittel zu diesem Zweck, umfassend die erfindungsgemäßen Phenoxyamidine. Weiterhin betrifft die Erfindung ein Verfahren zum Bekämpfen unerwünschter Mikroorganismen durch Ausbringen der erfindungsgemäßen Verbindungen auf die Mikroorganismen und/oder in deren Lebensraum.

WO-A-00/046 184 offenbart die Verwendung von Amidinen, insbesondere von N-Ethyl-N-methyl-N'-[4-(3-tertbutylylphenoxy)-2,5-dimethylphenyl]formamidin, als Fungizide.

WO-A-03/093 224 offenbart die Verwendung von Arylamidin-Derivaten als Fungizide.

WO-A-03/024 219 offenbart Fungizidzusammensetzungen umfassend wenigstens ein N2-Phenylamidin-Derivat in Kombination mit einem weiteren ausgewählten bekannten Wirkstoff.

WO-A-04/037239 offenbart antifungizide Medikamente auf der Basis von N2-Phenylamidin-Derivaten.

WO-A-05/089 547 offenbart Fungizidmischungen, umfassend wenigstens ein Arylamidin-Derivat in Kombination mit einem weiteren bekannten fungiziden Wirkstoff.

WO-A-05/120 234 offenbart Fungizidmischungen, umfassend wenigstens ein Phenylamidin-Derivat und ein weiteres ausgewähltes bekanntes Fungizid.

WO-A- 2007/031513 offenbart Thiazolyloxy substituierte Phenylamidin-Derivate als Fungizide.

Die Wirksamkeit der im Stand der Technik beschriebenen Amidine ist gut, lässt jedoch in manchen Fallen zu wünschen übrig.

Der vorliegenden Erfindung liegt daher die Aufgabe zugrunde, Amidine mit einer verbesserten fungiziden Wirksamkeit zur Verfügung zu stellen.

Die Aufgabe wurde überraschenderweise gelöst durch Fluoralkylphenylamidine der Formel (I) 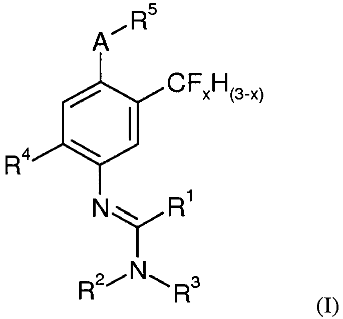 in welcher
- x: eine ganze Zahl, ausgewählt aus 1, 2 und 3 ist;
- A: ist O;
- R¹: ausgewählt ist aus Wasserstoff; linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₁₂-Alkenyl- oder C₄₋₁₂-Alkinyl - Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' Wasserstoff oder eine C₁₋₁₂₋Alkylgruppe sein kann;
X ein Halogenatom ist, das ausgewählt ist aus Fluor, Chlor, Brom und Iod;
-SH; -SR", wobei R" eine lineare oder verzweigte C₁₋₁₂-Alkylgruppe sein kann, die substituiert sein kann mit einer oder mehreren Gruppen, die ausgewählt sind aus - R', -X, -OR', -SR', -R'₂, -SiR'₃, -COOR', -CN und-CONR₂', worin R' die obigen Bedeutungen hat;
- R²: ausgewählt ist aus linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -NR'₂. -SiR'₃, -COOR', -CN und -CONR₂', substituiert sein können, wobei R' die obigen Bedeutungen hat;
- R³: ausgewählt ist aus -CN, -SH, -SR", -OR", -(C=O)-R", linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -R'₂, -SiR'₃, -COOR', -CN und -CONR₂', substituiert sein können, wobei R' und R" die obigen Bedeutungen haben;
- R⁴: ausgewählt ist aus der Gruppe bestehend aus -X, -CN, -SH, -SR", -OR", -(C=O)-R", linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Ar-alkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -R'₂, -SiR'₃, -COOR', -CN und -CONR₂', substituiert sein können, wobei R' und R" die obigen Bedeutungen haben;
ausgewählt ist aus 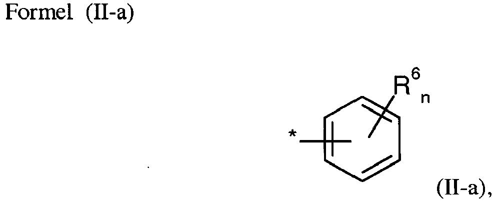 in der
n eine ganze Zahl von 0, 1, 2 oder 3 ist;
R⁶ ausgewählt ist aus Wasserstoff, linearen C₁₋₁₂-Alkyl-Gruppen, verzweigten oder cyclischen C₃₋₈-Alkyl-Gruppen, Halogenatomen, C₁₋₅-Haloalkyl-Gruppen; 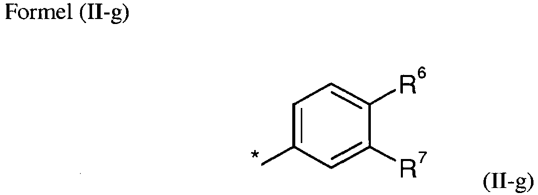 in welcher
R⁶ Halogen, eine C₁₋₈-Alkyl-Gruppe, eine C₁₋₅-Halogenalkyl-Gruppe ist und
R⁷ Wassersttoff, Halogen, Chlor oder Fluor, eine C₁₋₈-Alkyl-Gruppe oder eine C₁₋₅-Halogenalkyl-Gruppe ist,
oder R⁶ und R⁷ zusammen mit den Ringatomen, an die sie gebunden sind oder mit weiteren Atomen, ausgewählt aus N, O, P und S, einen vier- bis siebengliedrigen Ring bilden können, der mit einer oder mehreren X-, R'-, OR'-, SR'-, NR'₂-, SiR'₃-Gruppen substituiert sein kann, wobei R' die obigen Bedeutungen hat;
X ein Halogenatom ist, das ausgewählt ist aus Fluor, Chlor, Brom und Iod; 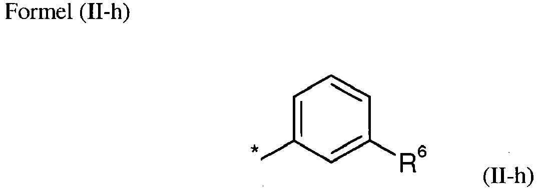 in welcher
R⁶ Halogen, eine lineare oder verzweigte C₁₋₈-Alkyl-Gruppe, eine C₃₋₁₂ Cycloalkyl-, C₅₋₁₈-Aryl-, C₇₋₁₉-Aralkyl- oder C₇₋₁₉-Alkaryl-Gruppen sein kann, wobei die zuvor genannten Gruppen mit SiR'₃-, -OR' und -CN substituiert sein können, wobei R' die obigen Bedeutungen hat ist;
und deren Salze.

Ein weiterer Gegenstand der vorliegenden Erfindung betrifft ein Verfahren zum Herstellen der erfindungsgemäßen Fluoralkylphenylamidine umfassend wenigstens einen der folgenden Schritte (a) bis (n):
(a) Umsetzung von Nitrobenzolderivaten der Formel (III) mit Alkoholen oder Phenolen der Formel (II) gemäß dem nachfolgenden Reaktionsschema: 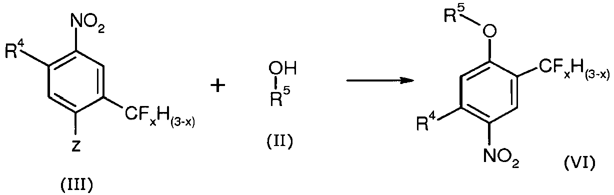
(b) Umsetzung von Nitrophenolderivaten der Formel (V) mit Phenylderivaten der Formel (IV) gemäß dem nachfolgenden Reaktionsschema: 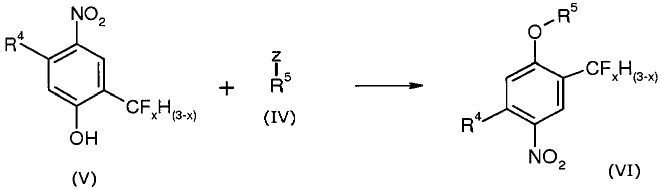
(c) Umsetzung von Anilinen der Formel (VII) mit Alkoholen oder Phenolen der Formel (II) gemäß dem nachfolgenden Reaktionsschema: 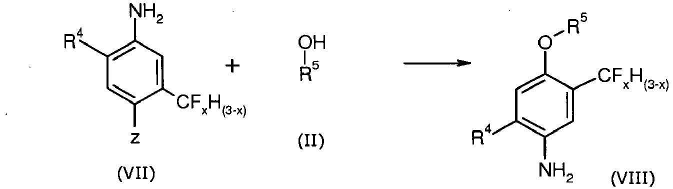
(d) Umsetzung von Aminophenolen der Formel (XII) mit Phenylderivaten der Formel (IV) gemäß dem nachfolgenden Reaktionsschema: 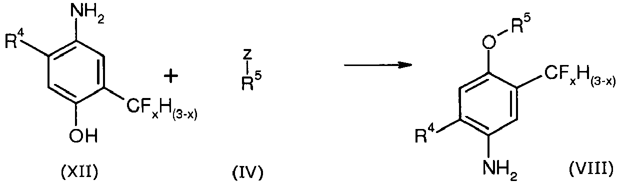
(e) Reduktion der Fluoralkylnitrophenole der Formel (VI) zu Fluoralkylanilinen der Formel (VIII) gemäß dem nachfolgenden Reaktionsschema: 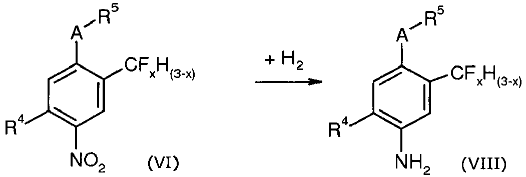
(f) Umsetzung der Anilinether der Formel (VIII) mit
   (i) Aminoacetalen der Formel (XIII) oder
   (ii) mit Amiden der Formel (XIV) oder
   (iii) mit Aminen der Formel (XV) in Gegenwart von Orthoestern der Formel (XVI) gemäß dem nachfolgenden Reaktionsschema: 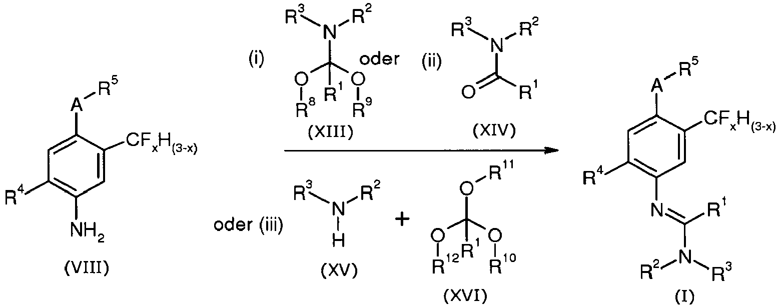
(g) Umsetzung der Aminophenole der Formel (XII) mit
   (i) Aminoacetalen der Formel (XIII) oder
   (ii) mit Amiden der Formel (XIV) oder
   (iii) mit Aminen der Formel (XV) in Gegenwart von Orthoestern der Formel (XVI) gemäß dem nachfolgenden Reaktionsschema: 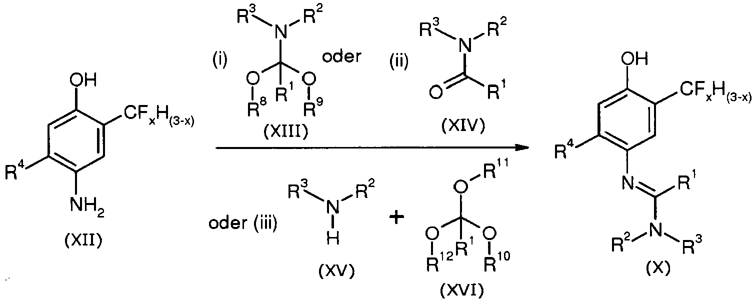
(h) Umsetzung der Aminophenole der Formel (VII) mit
   (i) Aminoacetalen der Formel (XIII) oder
   (ii) mit Amiden der Formel (XIV) oder
   (iii) mit Aminen der Formel (XV) in Gegenwart von Orthoestern der Formel (XVI) gemäß dem nachfolgenden Reaktionsschema: 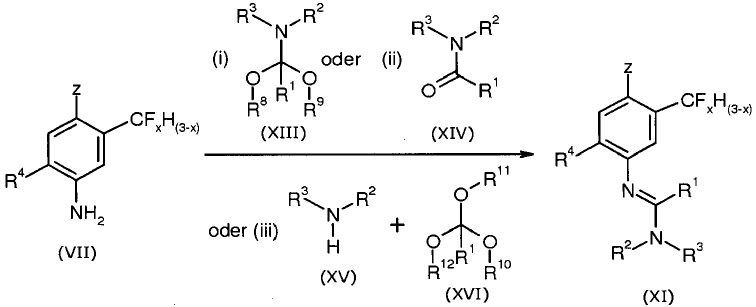
(i) Umsetzung von Amidinen der Formel (XI) mit Alkoholen oder Phenolen der Formel (II) gemäß dem nachfolgenden Reaktionsschema: 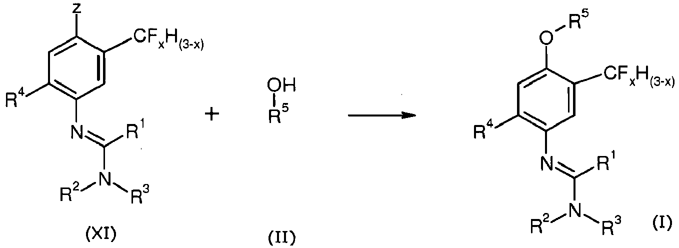
(j) Umsetzung von Amidinen der Formel (XI) mit Phenylderivaten der Formel (IV) gemäß dem nachfolgenden Reaktionsschema: 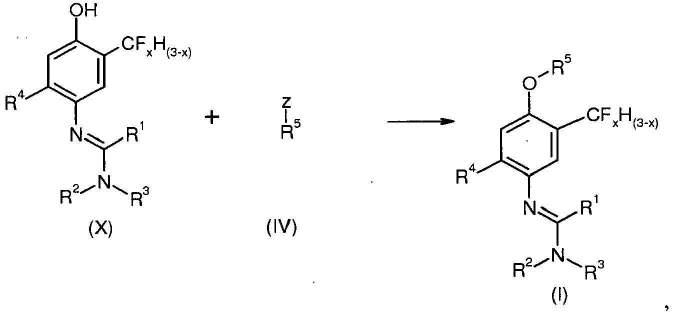
(k) Umsetzung von Fluoralkylanilinen der Formel (VII) mit Boronsäurederivaten der Formel (XVII) gemäß dem nachfolgenden Reaktionsschema: 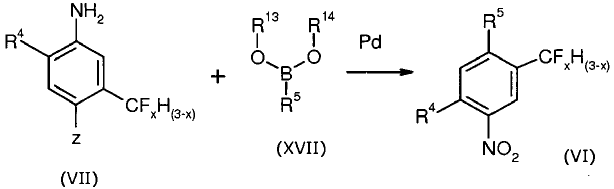
(l) Umsetzung von Fluoralkylanilinen der Formel (VII) mit Aldehyden oder Ketonen der Formel (XVIII) gemäß dem nachfolgenden Reaktionsschema: 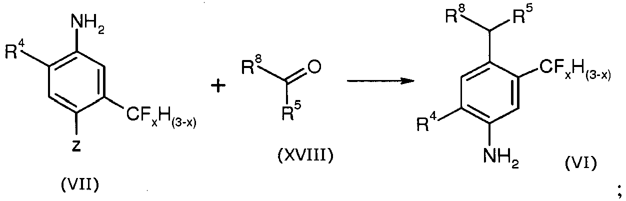
(m) Umsetzung von Fluoralkylnitrobenzolen der Formel (III) mit Boronsäurederivaten der Formel (XVII) gemäß dem nachfolgenden Reaktionsschema: 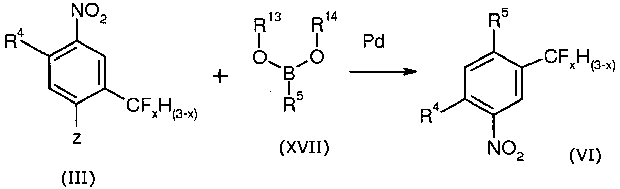
(n) Umsetzung von Fluoralkylnitrobenzolen der Formel (III) mit Aldehyden oder Ketonen der Formel (XVIII) gemäß dem nachfolgenden Reaktionsschema: 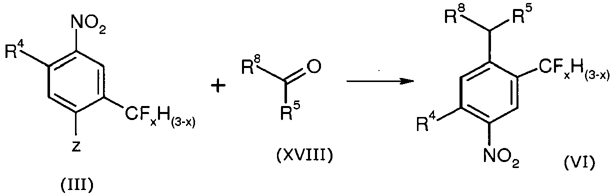 wobei in den zuvor beschriebenen Formeln
   - x, Z, R¹ bis R⁸: die obigen Bedeutungen haben;
   - R¹³ und R¹⁴: unabhängig voneinander ausgewählt sind aus der Gruppe bestehend aus Wasserstoff, C₁₋₁₂-Alkyl-, C₂₋₁₂-Alkenyl- C₂₋₁₂-AUdnyl- oder C₅₋₁₈-Aryl- oder C₇₋₁₉-Arylatkyl-Gruppen und die gemeinsam mit den O-Atomen, an die sie gebunden sind, einen fünf-, sechs- oder siebengliedrigen Ring bilden können.

Ein fünfter Gegenstand der Erfindung ist die nicht therapeutische Verwendung der erfindungsgemäßen Fluoralkylphenylamidine zum Bekämpfen unerwünschter Mikroorganismen. Ein sechster Gegenstand der vorliegenden Erfindung ist ein Mittel zum Bekämpfen unerwünschter Mikroorganismen, umfassend wenigstens ein Fluoralkylphenylamidin gemäß der vorliegenden Erfindung.

Ein weiterer Gegenstand der Erfindung betrifft ein nicht therapeutisches Verfahren zum Bekämpfen unerwünschter Mikroorganismen, dadurch gekennzeichnet, dass die erfindungsgemäßen Phenoxyamidine auf die Mikroorganismen und/oder in deren Lebensraum ausgebracht werden.

Außerdem betrifft die Erfindung ein Saatgut, welches mit wenigstens einem der erfindungsgemäßen Fluoralkylphenylamidine behandelt wurde.

Ein letzter Gegenstand der Erfindung betrifft ein Verfahren zum Schutz von Saatgut vor unerwünschten Mikroorganismen durch Verwendung eines mit wenigstens einem Fluoralkylphenylamidine der vorliegenden Erfindung behandelten Saatgutes.

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Im Zusammenhang mit der vorliegenden Erfindung bezeichnet die Gruppe -X ein Halogenatom, das ausgewählt ist aus Fluor, Chlor, Brom und Iod, vorzugsweise Fluor, Chlor und Brom, besonders bevorzugt aus Fluor und Chlor.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige Kohlenwasserstoff-Gruppen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für einen AlkylGruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen Alkenyl-Gruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy-(-OR'), Thioether- oder Mercapto- (-SR'), Amino- (-NR'₂), Silyl- (-SiR'₃), Carboxyl-(-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für einen Aryl-Gruppe mit 5 bis 18 Atomen. Im Einzelnen umfasst diese Definition beispielsweise die Bedeutungen Cyclopentadienyl, Phenyl, Cycloheptatrienyl, Cyclooctatetraenyl, Naphthyl und Anthracenyl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Die erfindungsgemäßen Fluoralkylphenylamidine sind Verbindungen der Formel (I) 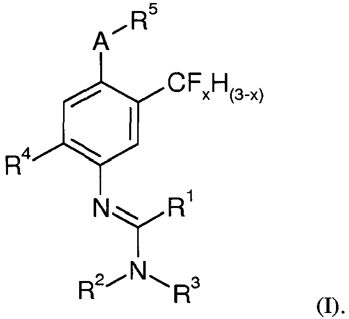 oder deren Salze, N-Oxide, Metallkomplexe und deren Stereoisomere.

In Formel (I) haben die Gruppen die im Folgenden definierten Bedeutungen. Die getroffenen Definitionen gelten für alle Zwischenprodukte gleichermaßen:
- x: eine ganze Zahl, ausgewählt aus 1, 2 und 3 ist;
- A: ausgewählt ist aus O, NH, CH₂, CHR⁸ und einer Einfachbindung;
- R¹: ist ausgewählt aus:
- Wasserstoff;
- linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₁₂-Alkenyl- oder C₄₋₁₂-Alkinyl -Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyano- (-CN) und Amid-Gruppen (-CONR₂'), substituiert sein können, wobei R' Wasserstoff oder eine C₁₋₁₂-Alkyl-Gruppe, vorzugsweise C₂₋₁₀-AlkylGruppe, besonders bevorzugt C₃₋₈-Alkyl-Gruppe sein kann;
   X ein Halogenatom ist, das ausgewählt ist aus Fluor, Chlor, Brom und Iod;
- Mercapto- (-SH) und Thioether-Gruppen (-SR"), wobei R" eine lineare oder verzweigte C₁₋₁₂-Alkyl-Gruppe, vorzugsweise C₂₋₁₀-Alkyl-Gruppe, besonders bevorzugt C₃₋₈-Alkyl-Gruppe sein kann, die substituiert sein kann mit Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyano- (-CN) und Amid-Gruppen (-CONR₂'), wobei R' die obigen Bedeutungen hat.
- R²: ist ausgewählt aus:
- linearen oder verzweigten C₁₋₁₂-Alkyl-, C₂₋₁₂-Alkenyl-, C₂₋₁₂-Alkinyl-Gruppen;
- cyclischen C₃₋₈-Alkyl-, C₄₋₈-Alkenyl-, C₄₋₈-Alkinyl-Gruppen;
- C₅₋₁₈-Aryl-, C₇₋₁₉-Aralkyl- oder C₇₋₁₉-Alkaryl-Gruppen,
wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl-(-COOR'), Cyano- (-CN) und Amid-Gruppen (-CONR₂'), substituiert sein können, wobei R' die obigen Bedeutungen hat.
- R³: ist ausgewählt aus:
- Cyano- (-CN), Mercapto- (-SH), Thioether- (-SR"), Alkoxy- (-OR") und AcylGruppen (-(C=O)-R"), wobei R' die obigen Bedeutungen hat;
- linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyano- (-CN) und Amid-Gruppen (-CONR₂'), substituiert sein können, wobei R' die obigen Bedeutungen hat;
- R⁴: ist ausgewählt aus:
- Halogenatomen (X-);
- Cyano- (-CN), Mercapto- (-SH), Thioether- (-SR"), Alkoxy- (-OR") und AcylGruppen (-(C=O)-R"), wobei R", die obigen Bedeutungen hat;
- linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyano- (-CN) und Amid-Gruppen (-CONR₂') substituiert sein können, wobei R' die obigen Bedeutungen hat.
- R⁵: ist ausgewählt aus: 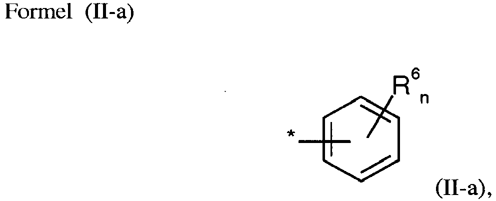 in der
n eine ganze Zahl von 0, 1, 2 oder 3 ist;
R⁶ ausgewählt ist aus Wasserstoff, linearen C₁₋₁₂-Alkyl-Gruppen, verzweigten oder cyclischen C₃₋₈-Alkyl-Gruppen, Halogenatomen, C₁₋₅-Haloalkyl-Gruppen; 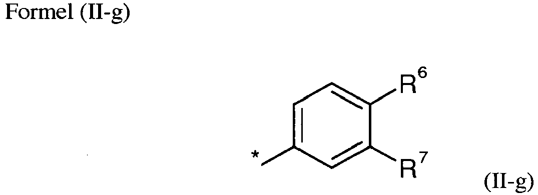 in welcher
R⁶ Halogen, eine C₁₋₈-Alkyl-Gruppe, eine C₁₋₅-Halogenalkyl-Gruppe ist und
R⁷ Wasserstoff, Halogen, Chlor oder Fluor, eine C₁₋₈-Alkyl-Gruppe oder eine C₁₋₅-Halogenalkyl-Gruppe ist,
oder R⁶ und R⁷ zusammen mit den Ringatomen, an die sie gebunden sind oder mit weiteren Atomen, ausgewählt aus N, O, P und S, einen vier- bis siebengliedrigen Ring bilden können, der mit einer oder mehreren X-, R'-, OR'-, SR'-, NR'₂-, SiR'₃-Gruppen substituiert sein kann, wobei R' die obigen Bedeutungen hat;
X ein Halogenatom ist, das ausgewählt ist aus Fluor, Chlor, Brom und Iod; 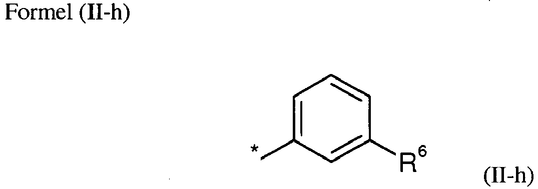 in welcher
R⁶ Halogen, eine lineare oder verzweigte C₁₋₈-Alkyl-Gruppe, eine C₃₋₁₂ Cycloalkyl-, C₅₋₁₈-Aryl-, C₇₋₁₉-Aralkyl- oder C₇₋₁₉-Alkaryl-Gruppen sein kann, wobei die zuvor genannten Gruppen mit SiR'₃-, -OR' und -CN substituiert sein können, wobei R' die obigen Bedeutungen hat ist.

In Formel (I) haben die Reste die im Folgenden definierten *bevorzugten* Bedeutungen. Die als *bevorzugt* getroffenen Definitionen gelten für alle Zwischenprodukte gleichermaßen:
- A: ist O;
- x: ist vorzugsweise eine ganze Zahl, ausgewählt aus 2 oder 3 ist;
- R¹: ist *vorzugsweise* ausgewählt aus der Gruppe bestehend aus Wasserstoff, einer Mercapto-Gruppe (-SH) oder C₁₋₈-Alkyl-Gruppen.
- R²: ist *vorzugsweise* ausgewählt aus linearen oder verzweigten C₁₋₈-ALkyl-Gruppen.
- R³: ist *vorzugsweise* ausgewählt aus linearen, verzweigten C₁₋₈-Alkyl- und cyclischen C₃₋₈-Alkyl-Gruppen.
- R⁴: ist *vorzugsweise* ausgewählt aus:
- Halogenatomen (-X);
- linearen oder verzweigten C₁₋₈-Alkyl-Gruppen oder
- linearen oder verzweigten C₁₋₅-Halogenalkyl-Gruppen.
- R⁵: ist *vorzugsweise* ausgewählt aus der Formel (II-a) 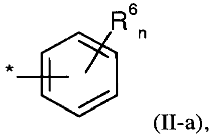 in der
* die Bindung zum A-Atom oder die Einfachbindung kennzeichnet.
n eine ganze Zahl von 0, 1, 2 oder 3 ist;
R⁶ ausgewählt ist aus Wasserstoff, linearen C₁₋₁₂-Alkyl-Gruppen, verzweigten oder cyclischen C₃₋₈-Alkyl-Gruppen, Halogenatomen, C₁₋₅-Haloalkyl-Gruppen;

In einer bevorzugten erfindungsgemäßen Ausführungsform ist A=O und in Gruppe (II-a) ist n=0.

In einer weiteren bevorzugten Ausführungsform der Erfindung ist A=0 und in Gruppe (II-a) ist n=2.

In Formel (I) haben die Reste die im Folgenden definierten *besonders bevorzugten* Bedeutungen. Die als *besonders bevorzugt* getroffenen Definitionen gelten für alle Zwischenprodukte gleichermaßen:
- A: ist Sauerstoff.
- x: ist *besonders* bevorzugt eine ganze Zahl, ausgewählt aus 2 oder 3 ist;
- R¹: ist *besonders bevorzugt* ausgewählt aus
- Wasserstoff,
- Methyl und Ethyl.
- R²: ist *besonders bevorzugt* ausgewählt aus Methyl und Ethyl.
- R³: ist besonders bevorzugt ausgewählt aus Methyl, Ethyl und Cyclopropyl.

Alternativ bilden R² und R³ besonders bevorzugt gemeinsam mit dem N-Atom, an das sie geknüpft sind einen Piperidyl- oder Pyrrolidyl-Ring.
- R⁴: ist *besonders bevorzugt* ausgewählt aus Cl- und F-Atomen und -CF₃, -CF₂H und Methyl-Gruppen.
und R⁵ ausgewählt ist aus den Gruppe (i) bis (ii)

### (i) Gruppen der Formel (II-g)

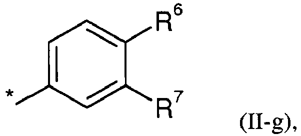

in welcher
- R⁶: Halogen, eine C₁₋₈-Alkyl-Gruppe, eine C₁₋₅-Halogenalkyl-Gruppe ist und
- R⁷: Wassersttoff, Halogen, Chlor oder Fluor, eine C₁₋₈-Alkyl-Gruppe oder eine C₁₋₅-Halogenalkyl-Gruppe ist,
oder R⁶ und R⁷ zusammen mit den Ringatomen, an die sie gebunden sind oder mit weiteren

Atomen, ausgewählt aus N, O, P und S, einen vier- bis siebengliedrigen Ring bilden können, der mit einer oder mehreren X-, R'-, OR'-, SR'-, N-R'₂-, SiR'₃-Gruppen substituiert sein kann, wobei R' die obigen Bedeutungen hat;
- X: ein Halogenatom ist, das ausgewählt ist aus Fluor, Chlor, Brom und Iod

### (ii) Gruppen der Formel (II-h)

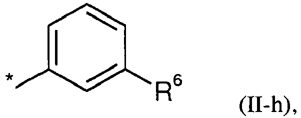

in welcher
- R⁶: Halogen, eine lineare oder verzweigte C₁₋₈-Alkyl-Gruppe, eine C₃₋₁₂ Cycloalkyl-, C₅₋₁₈-Aryl-, C₇₋₁₉-Aralkyl- oder C₇₋₁₉-Alkaryl-Gruppen sein kann, wobei die zuvor genannten Gruppen mit SiR'₃-, -OR' und -CN substituiert sein können, wobei R' die obigen Bedeutungen hat ist,

*Besonders bevorzugt* ist die erfindungsgemäße Ausführungsform bei der A=O und in Gruppe (II-g) R⁶= Cl oder F ist und R⁷ eine C₁₋₈ Alkylgruppe, vorzugsweise tert-Butyl oder iso-Propyl ist.

Weiterhin bevorzugt ist die Gruppe (II-h) im Zusammenhang mit A=O.

Je nach Art der oben definierten Substituenten weisen die erfindungsgemäßen Amidine saure oder basische Eigenschaften auf und können mit anorganischen oder organischen Säuren oder mit Basen oder mit Metallionen Salze, gegebenenfalls auch innere Salze oder Addukte bilden.

Als Metallionen kommen insbesondere die Ionen der Elemente der zweiten Hauptgruppe, insbesondere Calcium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigen-schaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Tragen die Verbindungen der Formeln (I) Amino, Alkylamino oder andere, basische Eigenschaften induzierende Gruppen, so können diese Verbindungen mit Säuren zu Salzen umgesetzt werden.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkyl-Gruppen mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkyl-Gruppen mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Die so erhältlichen Salze weisen ebenfalls fungizide Eigenschaften auf.

Im Zusammenhang mit der vorliegenden Erfindung besonders bevorzugte Amidine, sind ausgewählt aus der Gruppe bestehend aus (1) N-Ethyl-N-methyl-N'-[4-(3-*tert*-butyl-4-chlorophenoxy)-5-fluormethyl-2-methylphenyl]formamidine, (2) N-Ethyl-N-methyl-N'-[4-(4-*tert*-butyl-phenyl)-5-fluormethyl-2-methylphenyl]formamidine, (3) N-Ethyl-N-methyl-N'-[4-(3-*tert*-butyl-4-chlorophenoxy)-5-trifluormethyl-2-methylphenyl]formamidine, (4) N-Ethyl-N-methyl-N'-[4-(3-*iso*-propyl-phenoxy)-5-trifluormethyl-2-methylphenyl]formamidine, (5) N-Ethyl-N-methyl-N'-[4-(3-trifluormethyl-4-chlorophenoxy)-5-trifluormethyl-2-methylphenyl]-formamidine, (6) N-Propyl-N-methyl-N'-[4-(3-trifluormethyl-4-chlorophenoxy)-5-trifluor-methyl-2-methylphenyl]formamidine, , (8) N,N-Dimethyl-N'-[4-(3-*tert*-butyl-4-chlorophenoxy)-5-trifluormethyl-2-methylphenyl]-formamidine, (9) N-Ethyl-N-methyl-N'-[4-(3-*tert*-butyl-4-chlorophenoxy)-5-difluormethyl-2-methylphenyl]formamidine (10) N-Ethyl-N-methyl-N'-[4-(3,4-dichlorophenoxy)-5-difluormethyl-2-methylphenyl]formamidine, (11) N-Ethyl-N-methyl-N'-[4-(3-*tert*-butyl-phenoxy)-5-trifluormethyl-2-methylphenyl]formamidine, (12) N-Ethyl-N-methyl-N'-[4-(4-*tert*-butyl-phenyl)-5-trifluormethyl-2-ethylphenyl]form-amidine, (13) N-Ethyl-N-methyl-N'-[4-phenoxy-5-difluormethyl-2-methylphenyl]formamidine, (15) N-Ethyl-N-methyl-N'-{4-[3-(1-hydroxyethyl)-phenoxy]-5-difluormethyl-2-methylphenyl}formamidine, (16) N-Ethyl-N-methyl-N'-{4-[3-(1-fluorethyl)-phenoxy]-5-difluormethyl-2-methylphenyl}formamidine, (17) N-Ethyl-N-methyl-N'-[4-(3-*iso*-propyl-4-chlorophenoxy)-5-difluormethyl-2-methylphenyl]formamidine, (18) N-Ethyl-N-methyl-N'-[4-(3-*iso*-propyl-phenoxy)-5-difluormethyl-2-methylphenyl]formamidine.

### Herstellung der erfindungsgemäßen Amidine

Die erfindungsgemäßen Amidine können durch das im folgenden Schema (I) dargestellte Verfahren erhalten werden:

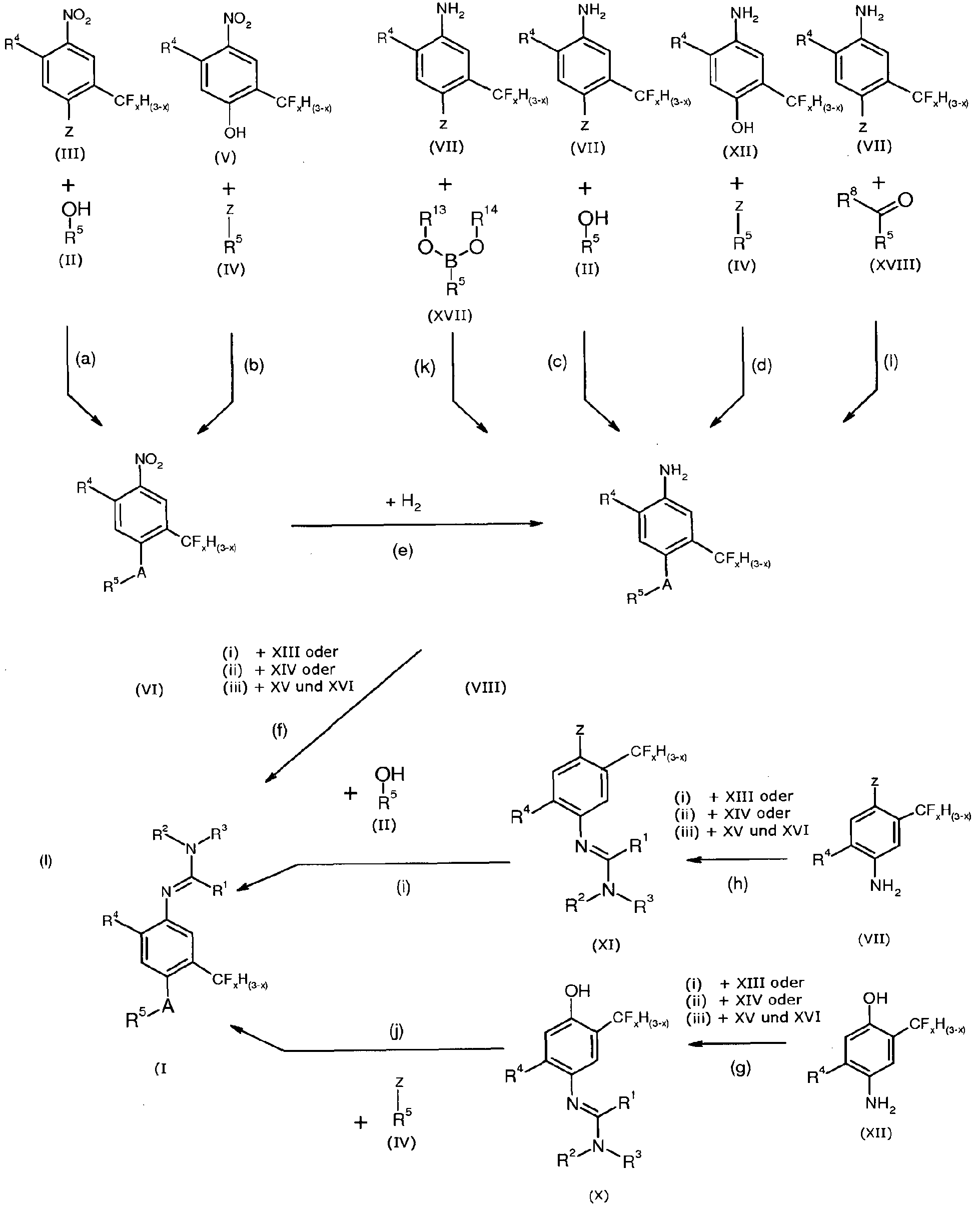

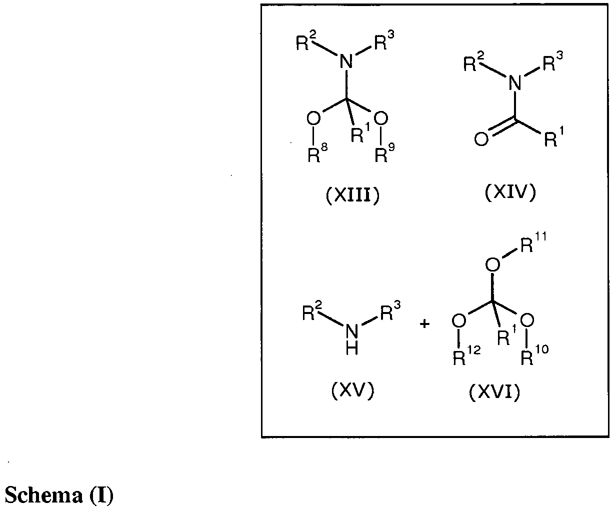

### Schritt (a)

In einer erfindungsgemäßen Ausführungsform werden Nitrobenzolderivate der Formel (III) mit Alkoholen oder Phenolen der Formel (II) oder den aus diesen gebildeten Alkoholaten oder Phenolaten gemäß dem nachfolgenden Reaktionsschema zu Nitrophenylethern der Formel (VI) umgesetzt:

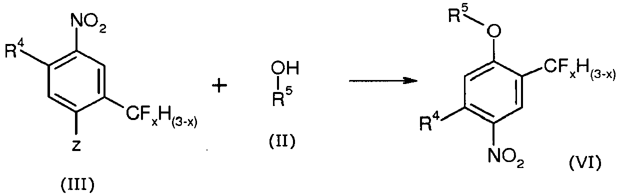

Als Austrittsgruppe (Z) sind alle Substituenten geeignet, die bei den vorherrschenden Reaktionsbedingungen eine ausreichende Nucleofugizität aufweisen. Beispielhaft seien Halogene, Triflat, Mesylat, Tosylat oder SO₂Me als geeignete Austrittsgruppen genannt.

Die Reaktion erfolgt ggf. in Gegenwart einer Base.

Geeignete Basen sind organische und anorganische Basen, die üblicherweise in solchen Reaktionen verwendet werden. Vorzugsweise werden Basen verwendet, die beispielhaft ausgewählt sind aus der Gruppe bestehend aus Hydriden, Hydroxiden, Amiden, Alkoholaten, Acetaten, Fluoriden, Phosphaten, Carbonaten und Hydrogencarbonaten von Alkali- oder Erdalkalimetallen. Besonders bevorzugt sind dabei Natriumamid, Natriumhydrid, Lithiumdiisopropylamid, Natriummethanolat, Kalium-*tert*-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat. Außerdem sind tertiäre Amine, wie z.B. Trimethylamin, Triethylamin, Tributylamin, N,N-Dimethylanilin, N,N-Dimethylbenzylamin, Pyridin, N-Methylpiperidin, N-Methylpyrolidon, N,N-Dimethylaminopyridin, Diazabicyclooctan (DABCO), Diazabicyclononen (DBN) und Diazabicycloundecen (DBU).

Gegebenenfalls kann ein Katalysator, der ausgewählt ist aus der Gruppe bestehend aus Palladium, Kupfer und deren Salzen oder Komplexen eingesetzt werden.

Die Reaktion des Nitrobenzolderivats mit dem Phenol kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln.

Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; oder Mischungen dieser mit Wasser sowie reines Wasser.

Die Reaktion kann im Vakuum, bei Normaldruck oder unter Überdruck und bei Temperaturen von -20 bis 200 °C durchgeführt werden, vorzugsweise erfolgt die Reaktion bei Normaldruck und Temperaturen von 50 bis 150 °C.

### Schritt (b)

In einer alternativen erfindungsgemäßen Ausführungsform werden Nitrophenolderivate der Formel (V) oder die daraus gebildeten Phenolate mit Phenylderivaten der Formel (IV) gemäß dem nachfolgenden Reaktionsschema zu Nitrophenylethern der Formel (VI) umgesetzt:

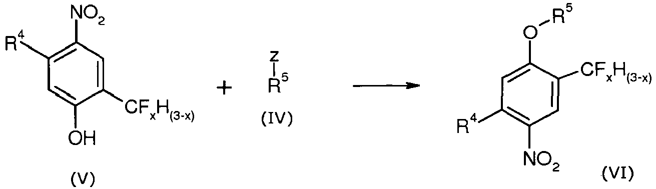

Im Hinblick auf Reaktionsbedingungen, Lösungsmittel, Katalysatoren und geeignete AustrittsGruppen sei auf Schritt (a) verwiesen.

### Schritt (c)

In einer weiteren alternativen erfindungsgemäßen Ausführungsform werden Aniline der Formel (VII) mit Alkoholen oder Phenolen der Formel (II) oder den aus diesen gebildeten Alkoholaten oder Phenolaten gemäß dem nachfolgenden Reaktionsschema zu Aminophenylethern der Formel (VIII) umgesetzt:

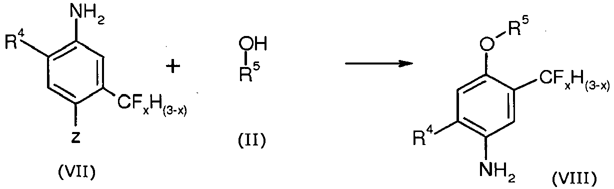

Im Hinblick auf Reaktionsbedingungen, Lösungsmittel, Katalysatoren und geeignete AustrittsGruppen sei auf Schritt (a) verwiesen.

### Schritt (d)

In einer weiteren alternativen erfindungsgemäßen Ausführungsform werden Aminophenole der Formel (XII) mit Phenylderivaten der Formel (IV) gemäß dem nachfolgenden Reaktionsschema zu Aminophenylethern der Formel (VIII) umgesetzt:

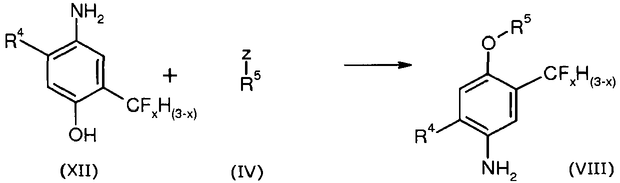

Im Hinblick auf Reaktionsbedingungen, Lösungsmittel, Katalysatoren und geeignete AustrittsGruppen sei auf Schritt (c) verwiesen.

### Schritt (e)

Die in den Schritten (a), (b) und (n) erhaltenen Fluoralkylnitrophenole der Formel (VI) können gemäß dem nachfolgenden Reaktionsschema zu den Fluoralkylanilinen der Formel (VIII) reduziert werden:

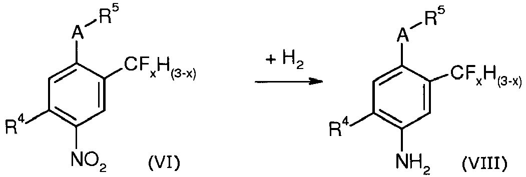

Die Reduktion gemäß Schritt (e) kann durch sämtliche im Stand der Technik zur Reduktion von Nitro-Gruppen beschriebenen Methoden erfolgen.

Ggf. erfolgt die Reduktion mit Zinnchlorid in konzentrierter Salzsäure wie in WO 0046184 beschrieben. Alternativ kann die Reduktion aber auch mit Wasserstoffgas, vorzugsweise in Gegenwart geeigneter Hydrierkatalysatoren, wie z.B. Raney Nickel, Pd/C erfolgen. Die Reaktionsbedingungen sind im Stand der Technik vorbeschrieben und dem Fachmann geläufig.

Wird die Reduktion in der Flüssigphase durchgeführt, so sollte die Reaktion in einem gegenüber den vorherrschenden Reaktionsbedingungen inerten Lösungsmittel erfolgen. Ein solches ist beispielsweise Toluol.

### Schritt (f)

Die Umsetzung der Anilinethern der Formel (VIII) zu den erfindungsgemäßen Amidinen der Formel (I) gemäß Schritt (f) kann, wie zuvor in Schema (I) dargestellt nach unterschiedlichen alternativen Methoden unter Verwendung von
(i) Aminoacetalen der Formel (XIII) oder
(ii) Amiden der Formel (XIV) oder
(iii) Aminen der Formel (XV) in Gegenwart von Orthoestern der Formel (XVI)
gemäß dem nachfolgenden Reaktionsschema erfolgen:

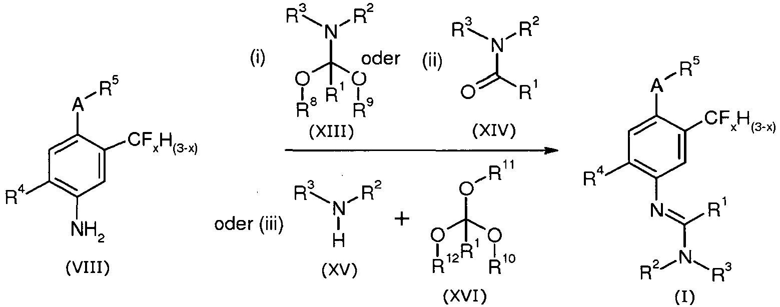

Die einzelnen alternativen Ausführungsformen (i) bis (iii) des erfindungsgemäßen Verfahrens sollen infolge kurz erläutert werden:
(i) Gemäß einer erfindungsgemäßen Ausführungsform, die im Schema (I) als Schritt (i) dargestellt ist, werden die Anilinether der Formel (VIII) mit Aminoacetalen der Formel (XIII), in der R² und R³ wie zuvor beschrieben definiert sind und R⁸ und R⁹ ausgewählt sind aus C₁₋₈-Alkyl-Gruppen, vorzugsweise aus C₂₋₆-Alkyl-Gruppen, besonders bevorzugt aus C₃₋₅-Alkyl-Gruppen und die gemeinsam mit den O-Atomen, an die sie gebunden sind, einen fünf- oder sechsgliedrigen carbocyclischen Ring bilden können, zu den erfindungsgemäßen Phenoxyphenylamidinen der Formel (I) umgesetzt.

Die Aminoacetale der Formel (XIII) sind aus den in JACS, 65, 1566 (1943) beschriebenen Formamiden durch Umsetzung mit Alkylierungsreagenzien, wie z.B. Dimethylsulfat, erhältlich.

Die Reaktion gemäß Schritt (i) erfolgt ggf. in Gegenwart einer Säure.

Geeignete Säuren sind beispielsweise ausgewählt aus der Gruppe bestehend aus organischen und anorganischen Säuren, wobei p-Toluolsulfonsäure, Methansulfonsäure, Salzsäure (gasförmig, wässrig oder in organischer Lösung) oder Schwefelsäure.
(ii) In einer alternativen erfindungsgemäßen Ausführungsform, die im Schema (I) als Schritt (ii) dargestellt ist, werden die Anilinether der Formel (VIII) mit Amiden der Formel (XIV), in der die Gruppen R¹ bis R³ wie zuvor definiert sind, zu den erfindungsgemäßen Phenoxyphenylamidinen umgesetzt.

Die Reaktion gemäß Schritt (ii) erfolgt ggf. in Gegenwart eines Halogenierungsmittels. Geeignete Halogenierungsmittel sind beispielsweise ausgewählt aus der Gruppe bestehend aus PCl₅, PCl₃, POCl₃ oder SOCl₂.

Zudem kann die Reaktion alternativ in Gegenwart eines Kondensationsmittels erfolgen.

Geeignete Kondensationsmittel sind solche, die üblicherweise zur Knüpfung von Amidbindungen verwendet werden, beispielhaft seien Säurehalogenidbildner wie z.B. Phosgen, Phosphortribromid, Phosphortrichlorid, Phosphorpentachlorid, Phosphortrichloridoxid oder Thionylchlorid; Anhydridbildner wie z.B. Chlorformiat, Methylchlorformiat, Isopropylchlorformiat, Isobutylchlorformiat oder Methansulfonylchlorid; Carbodiimide wie z.B. N,N'-Dicyclohexylcarbodiimin (DCC) oder andere übliche Kondensationsmittel wie z.B. Phosphorpentoxid, Polyphosphorsäure, N,N'-Carbodiimidazol, 2-Ethoxy-N-ethoxycarbonyl-1,2-dihydroquinolin (EEDQ), Triphenylphosphin/Tetrachlormethan oder Bromtripyrrolidinophosphoniumhexafluorophosphat genannt.

Die Reaktion gemäß Schritt (ii) erfolgt ggf. in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxiethanol, Methoxyethanol, Diethylenglycolmonomethylether, Diethylenglycolmonoethylether oder Mischungen dieser verwendet.
(iii) Gemäß einer weiteren alternativen erfindungsgemäßen Ausführungsform, die in Schema (I) als Schritt (iii) dargestellt ist, werden die Anilinether der Formel (VIII) mit Aminen der Formel (XV), in der die Gruppen R² und R³ wie zuvor definiert sind in Gegenwart von Orthoestern der Formel (XVI), in der R¹ wie zuvor definiert ist und R¹⁰ bis R¹² unabhängig voneinander ausgewählt sind aus C₁₋₈-Alkyl-Gruppen, vorzugsweise aus C₂₋₆-Alkyl-Gruppen, besonders bevorzugt aus C₃₋₅-Alkyl-Gruppen, zu den erfindungsgemäßen 4-substituierten Phenoxyphenylamidinen umgesetzt.

Die Reaktion gemäß Schritt (iii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxiethanol, Methoxyethanol, Diethylenglycolmonomethylether, Diethylenglycolmonoethylether; oder Mischungen dieser mit Wasser sowie reines Wasser verwendet.

### Schritt (g)

In einer alternativen erfindungsgemäßen Ausführungsform können bereits die Aminophenole der Formel (XII)
(i) mit Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema zu Amidinen der Formel (X) umgesetzt werden:

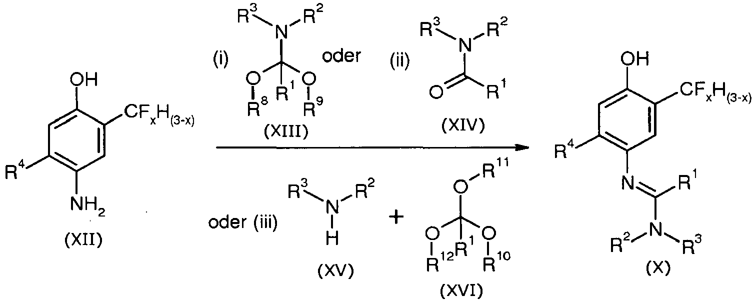

Hinsichtlich der Reaktionsbedingungen, Lösungsmittel, Katalysatoren sei auf Schritt (f) verwiesen.

Die weitere Umsetzung der Amidine der Formel (X) zu den erfindungsgemäßen Zielmolekülen der Formel (I) kann beispielsweise wie in Schritt (j) beschrieben erfolgen.

### Schritt (h)

In einer alternativen erfindungsgemäßen Ausführungsform können die Aminophenylderivate der Formel (VII)
(i) mit Aminoacetalen der Formel (XIII) oder
(ii) mit Amiden der Formel (XIV) oder
(iii) mit Aminen der Formel (XV) in Gegenwart von Orthoestern der Formel (XVI) gemäß dem nachfolgenden Reaktionsschema zu Amidinen der Formel (XI) umgesetzt werden:

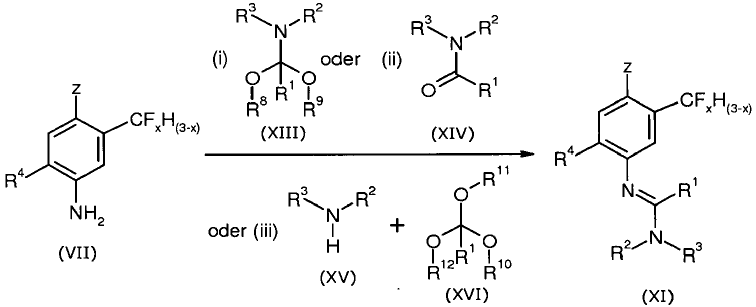

Hinsichtlich der Reaktionsbedingungen, Lösungsmittel, Katalysatoren sei auf Schritt (f) verwiesen.

Die weitere Umsetzung der Amidine der Formel (XI) zu den erfindungsgemäßen Zielmolekülen der Formel (I) kann beispielsweise wie in Schritt (i) beschrieben erfolgen.

### Schritt (i)

Gemäß einer weiteren erfindungsgemäßen Ausführungsform können die aus Schritt (h) erhältlichen Amidine der Formel (XI) mit Alkoholen oder Phenolen der Formel (II) oder den aus diesen gebildeten Alkoholaten oder Phenolaten zu den erfindungsgemäßen Zielmolekülen der Formel (I) gemäß dem nachfolgenden Reaktionsschema umgesetzt werden:

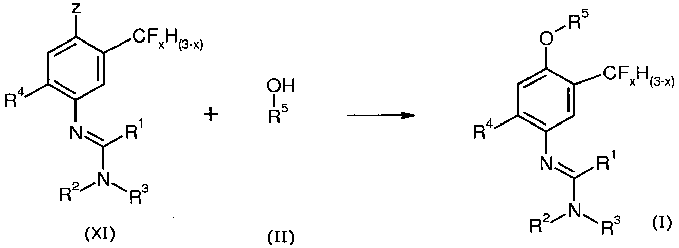

Hinsichtlich der Reaktionsbedingungen, Lösungsmittel und Katalysatoren sei auf Schritt (a) verwiesen.

### Schritt (j)

Gemäß einer weiteren erfindungsgemäßen Ausführungsform können die aus Schritt (g) erhältlichen Amidine der Formel (X) mit 4-substituierten Phenylderivaten der Formel (IV) zu den erfindungsgemäßen Zielmolekülen der Formel (I) gemäß dem nachfolgenden Reaktionsschema umgesetzt werden:

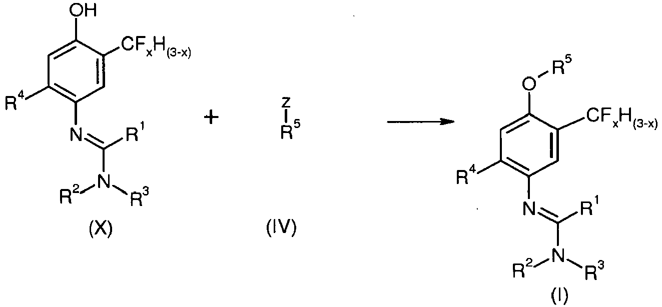

Hinsichtlich der Reaktionsbedingungen, Lösungsmittel und Katalysatoren sei auf Schritt (b) verwiesen.

### Schritt (k)

Die Umsetzung von Fluoralkylanilinen der Formel (VII) mit Boronsäurederivaten der Formel (XVII) gemäß dem nachfolgenden Reaktionsschema:

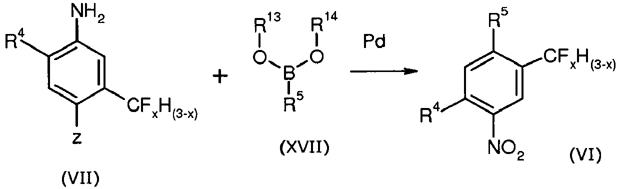

In den zuvor beschriebenen Formeln haben
x, Z, R¹ bis R⁵ die obigen Bedeutungen
R¹³ und R¹⁴ sind unabhängig voneinander ausgewählt sind aus der Gruppe bestehend aus Wasserstoff, C₁₋₁₂-Alkyl-, C₂₋₁₂-Alkenyl- C₂₋₁₂-Alkinyl- oder C₅₋₁₈-Aryl- oder C₇₋₁₉-Arylalkyl-Gruppen und können die gemeinsam mit den O-Atomen, an die sie gebunden sind, einen fünf-, sechs- oder siebengliedrigen Ring bilden.

Die Umsetzung erfolgt nach der Suzuki-Methode, die in PCT/EP2006/066271 näher erläutert ist.

### Schritt (l)

Umsetzung von Fluoralkylanilinen der Formel (VII) mit Aldehyden oder Ketonen der Formel (XVIII) gemäß dem nachfolgenden Reaktionsschema:

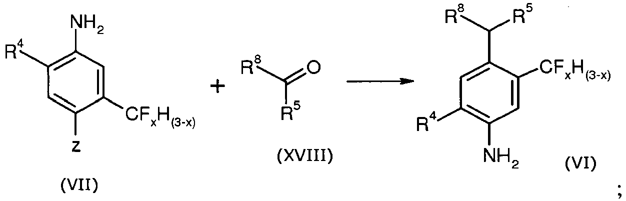

Hinsichtlich der Verfahrensbedingungen sei auf die Offenbarung der PCT/EP2006/066295 verwiesen.

### Schritt (m)

Umsetzung von Fluoralkylnitrobenzolen der Formel (III) mit Boronsäurederivaten der Formel (XVII) gemäß dem nachfolgenden Reaktionsschema: 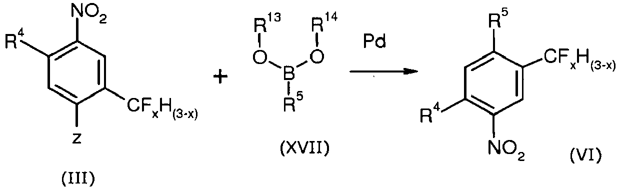 wobei in den zuvor beschriebenen Formeln

Hinsichtlich der Verfahrensbedingungen sei auf die Offenbarung der PCT/EP2006/066295 verwiesen.

### Schritt (n)

Umsetzung von Fluoralkylnitrobenzolen der Formel (III) mit Aldehyden oder Ketonen der Formel (XVIII) gemäß dem nachfolgenden Reaktionsschema:

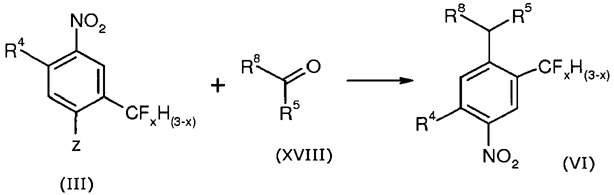

Die Umsetzung erfolgt nach der Suzuki-Methode, die in PCT/EP2006/066271 näher erläutert ist.

Im Zusammenhang mit den erfindungsgemäßen Verfahren zur Herstellung der Amidine der Formel (I) sind die folgenden Kombinationen von Reaktionsschritten als Vorteilhaft anzusehen: Schritte (a), (e) und (f); Schritte (k), (e) und (f); Schritte (m), (e) und (f); Schritte (b), (e) und (f); Schritte (c) und (f); Schritte (d) und (f); Schritte (l) und (f); Schritte (n) und (f); Schritte (h) und (i) und/oder Schritte (g) und (j).

Die Herstellung der erfindungsgemäßen Amidine erfolgt vorzugsweise ohne zwischenzeitliche Isolierung der Zwischenprodukte.

Die abschließende Reinigung der Amidine kann durch übliche Reinigungsverfahren erfolgen. Vorzugsweise erfolgt die Reinigung durch Kristallisation.

### Bekämpfung von unerwünschten Mikroorganismen

Die erfindungsgemäßen Amidine weisen eine starke mikrobizide Wirkung auf und können zur Bekämpfung von unerwünschten Mikroorganismen, wie Fungi und Bakterien, im Pflanzenschutz und im Materialschutz eingesetzt werden.

### Pflanzenschutz

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;
Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;
Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B.
Alternaria-Arten, wie beispielsweise Alternaria brassicicola
Aphanomyces-Arten, wie beispielsweise Aphanomyces euteiches
Ascochyta-Arten, wie beispielsweise Ascochyta lentis
Aspergillus-Arten, wie beispielsweise Aspergillus flavus
Cladosporium-Arten, wie beispielsweise Cladosporium herbarum
Cochliobolus-Arten, wie beispielsweise Cochliobolus sativus
(Konidienform: Drechslera, Bipolaris Syn: Helminthosporium);
Colletotrichum-Arten, wie beispielsweise Colletotrichum coccodes;
Fusarium-Arten, wie beispielsweise Fusarium culmorum;
Gibberella-Arten, wie beispielsweise Gibberella zeae;
Macrophomina-Arten, wie beispielsweise Macrophomina phaseolina
Monographella-Arten, wie beispielsweise Monographella nivalis;
Penicillium-Arten, wie beispielsweise Penicillium expansum
Phoma-Arten, wie beispielsweise Phoma lingam
Phomopsis-Arten, wie beispielsweise Phomopsis sojae;
Phytophthora Arten, wie beispielsweise Phytophthora cactorum;
Pyrenophora-Arten, wie beispielsweise Pyrenophora graminea
Pyricularia-Arten, wie beispielsweise Pyricularia oryzae;
Pythium-Arten, wie beispielsweise Pythium ultimum;
Rhizoctonia-Arten, wie beispielsweise Rhizoctonia solani;
Rhizopus-Arten, wie beispielsweise Rhizopus oryzae
Sclerotium-Arten, wie beispielsweise Sclerotium rolfsii;
Septoria-Arten, wie beispielsweise Septoria nodorum;
Typhula-Arten, wie beispielsweise Typhula incarnata;
Verticillium-Arten, wie beispielsweise Verticillium dahliae
Krebserkrankungen, Gallen und Hexenbesen, hervorgerufen durch z.B.
Nectria-Arten, wie beispielsweise Nectria galligena;
Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)
Pilzkrankheiten an Wurzeln und der Stängelbasis verursacht durch z.B.
Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraums nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

### Mycotoxine

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

### Materialschutz

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

### Formulierungen

Die vorliegende Erfindung betrifft ein Mittel zum Bekämpfen unerwünschter Mikroorganismen, umfassend wenigstens eines der erfindungsgemäßen Phenoxyamidine.

Die erfindungsgemäßen Phenoxyamidine können dazu in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die zuvor beschriebenen Formulierungen können in einem erfindungsgemäßen Verfahren zum Bekämpfen unerwünschter Mikroorganismen verwendet werden, bei dem die erfindungsgemäßen Phenoxyamidine auf die Mikroorganismen und/oder in deren Lebensraum ausgebracht werden.

### Saatgutbehandlung

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einem erfindungsgemäßen Mittel behandelt wird.

Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen.

Weiterhin bezieht sich die Erfindung auf Saatgut, welches zum Schutz vor phytopathogenen Pilzen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate. Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

### Mischung mit bekannten Fungiziden, Bakteriziden, Akariziden, Nernatiziden oder Insektiziden

Die erfindungsgemäßen Phenoxyamidine können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Beim Einsatz der erfindungsgemäßen Phenoxyamidine als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

### GMO's

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-StreßBedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben (WO 1992/005251, WO 1995/009910, WO 1998/27806, WO 2005/002324, WO 2006/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z. B. WO 1991/002069).

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., Science (1983), 221, 370-371), das CP4-Gen des Bakteriums Agrobacterium sp. (Barry et al., Curr. Topics Plant Physiol. (1992), 7, 139-145), die Gene, die für eine EPSPS aus der Petunie (Shah et al., Science (1986), 233, 478-481), für eine EPSPS aus der Tomate (Gasser et al., J. Biol. Chem. (1988), 263, 4280-4289) oder für eine EPSPS aus Eleusine (WO 2001/66704) kodieren. Es kann sich auch um eine mutierte EPSPS handeln, wie sie zum Beispiel in EP-A 0837944, WO 2000/066746, WO 2000/066747 oder WO 2002/026995 beschrieben ist. Glyphosatetolerante Pflanzen können auch dadurch erhalten werden, daß man ein Gen exprimiert, das für ein Glyphosate-Oxidoreduktase-Enzym, wie es in US 5,776,760 und US 5,463,175 beschrieben ist, kodiert. Glyphosatetolerante Pflanzen können auch dadurch erhalten werden, daß man ein Gen exprimiert, das für ein Glyphosateacetyltransferase-Enzym, wie es in z. B. WO 2002/036782, WO 2003/092360, WO 2005/012515 und WO 2007/024782 beschrieben ist, kodiert. Glyphosatetolerante Pflanzen können auch dadurch erhalten werden, daß man Pflanzen, die natürlich vorkommende Mutationen der oben erwähnten Gene, wie sie zum Beispiel in WO 2001/024615 oder WO 2003/013226 beschrieben sind, enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind zum Beispiel in US 5,561,236; US 5,648,477; US 5,646,024; US 5,273,894; US 5,637,489; US 5,276,268; US 5,739,082; US 5,908,810 und US 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym gemäß WO 1996/038567, WO 1999/024585 und WO 1999/024586 kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen und Gene sind in WO 1999/034008 und WO 2002/36787 beschrieben. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert, wie dies in WO 2004/024928 beschrieben ist.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies zum Beispiel bei Tranel und Wright, Weed Science (2002), 50, 700-712, jedoch auch in US 5,605,011, US 5,378,824, US 5,141,870 und US 5,013,659, beschrieben ist. Die Herstellung von sulfonylharnstofftoleranten Pflanzen und imidazolinontoleranten Pflanzen ist in US 5,605,011; US 5,013,659; US 5,141,870; US 5,767,361; US 5,731,180; US 5,304,732; US 4,761,373; US 5,331,107; US 5,928,937; und US 5,378,824; sowie in der internationalen Veröffentlichung WO 1996/033270 beschrieben. Weitere imidazolinontolerante Pflanzen sind auch in z. B. WO 2004/040012, WO 2004/106529, WO 2005/020673, WO 2005/093093, WO 2006/007373, WO 2006/015376, WO 2006/024351 und WO 2006/060634 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden, wie dies zum Beispiel für die Sojabohne in US 5,084,082, für Reis in WO 1997/41218, für die Zuckerrübe in US 5,773,702 und WO 1999/057965, für Salat in US 5,198,599 oder für die Sonnenblume in WO 2001/065922 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus Bacillus thuringiensis oder einen insektiziden Teil davon, wie die insektiziden Kristallproteine, die von Crickmore et al., Microbiology and Molecular Biology Reviews (1998), 62, 807-813, von Crickmore et al. (2005) in der Bacillus thuringiensis -Toxinnomenklatur aktualisiert, online bei: http://www.lifesci.sussex.ac.uk/Home/Neil_CrickmoreBt/), zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Moellenbeck et al., Nat. Biotechnol. (2001), 19, 668-72; Schnepf et al., Applied Environm. Microb. (2006), 71, 1765-1774); oder
3) ein insektizides Hybridprotein, das Teile von zwei unterschiedlichen insektiziden Kristallproteinen aus Bacillus thuringiensis umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus Bacillus thuringiensis oder Bacillus cereus oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 1994/21795).
7) ein insektizides Hybridprotein, das Teile von verschiedenen sezernierten Proteinen von Bacillus thuringiensis oder Bacillus cereus umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 2000/004173 oder EP 04077984.5 oder EP 06009836.5 beschrieben ist.
b. Pflanzen, die ein streßtoleranzförderndes Transgen enthalten, das die Expression und/oder Aktivität der für PARG kodierenden Gene der Pflanzen oder Pflanzenzellen zu reduzieren vermag, wie dies z.B. in WO 2004/090140 beschrieben ist;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase, wie dies z. B. in EP 04077624.7 oder WO 2006/133827 oder PCT/EP07/002433 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet. Diese transgenen Pflanzen, die eine modifizierte Stärke synthetisieren, sind zum Beispiel in EP 0571427, WO 1995/004826, EP 0719338, WO 1996/15248, WO 1996/19581, WO 1996/27674, WO 1997/11188, WO 1997/26362, WO 1997/32985, WO 1997/42328, WO 1997/44472, WO 1997/45545, WO 1998/27212, WO 1998/40503, WO 99/58688, WO 1999/58690, WO 1999/58654, WO 2000/008184, WO 2000/008185, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, EP 06090134.5, EP 06090228.5, EP 06090227.7, EP 07090007.1, EP 07090009.7, WO 2001/14569, WO 2002/79410, WO 2003/33540, WO 2004/078983, WO 2001/19975, WO 1995/26407, WO 1996/34968, WO 1998/20145, WO 1999/12950, WO 1999/66050, WO 1999/53072, US 6,734,341, WO 2000/11192, WO 1998/22604, WO 1998/32326, WO 2001/98509, WO 2001/98509, WO 2005/002359, US 5,824,790, US 6,013,861, WO 1994/004693, WO 1994/009144, WO 1994/11520, WO 1995/35026 bzw. WO 1997/20936 beschrieben.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, wie dies in EP 0663956, WO 1996/001904, Wo 1996/021023, WO 1998/039460 und WO 1999/024593 beschrieben ist, Pflanzen, die alpha-1,4-Glucane produzieren, wie dies in WO 1995/031553, US 2002/031826, US 6,284,479, US 5,712,107, WO 1997/047806, WO 1997/047807, WO 1997/047808 und WO 2000/14249 beschrieben ist, Pflanzen, die alpha-1,6-verzweigte alpha-1,4-Glucane produzieren, wie dies in WO 2000/73422 beschrieben ist, und Pflanzen, die Alternan produzieren, wie dies in WO 2000/047727, EP 06077301.7, US 5,908,975 und EP 0728213 beschrieben ist.
3) Transgene Pflanzen, die Hyaluronan produzieren, wie dies zum Beispiel in WO 2006/032538, WO 2007/039314, WO 2007/039315, WO 2007/039316, JP 2006/304779 und WO 2005/012529 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten, wie dies in WO 1998/000549 beschrieben ist,
b) Pflanzen wie Baumwollpflanzen, die eine veränderte Form von rsw2- oder rsw3-homologen Nukleinsäuren enthalten, wie dies in WO 2004/053219 beschrieben ist;
c) Pflanzen wie Baumwollpflanzen mit einer erhöhten Expression der Saccharosephosphatsynthase, wie dies in WO 2001/017333 beschrieben ist;
d) Pflanzen wie Baumwollpflanzen mit einer erhöhten Expression der Saccharosesynthase, wie dies in WO 02/45485 beschrieben ist;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 2005/017157 beschrieben ist;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen, wie dies in WO 2006/136351 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren, wie dies zum Beispiel in US 5,969,169, US 5,840,946 oder US 6,323,392 oder US 6,063, 947 beschrieben ist;
b) Pflanzen wie Rapspflanzen, die Öl mit einem niedrigen Linolensäuregehalt produzieren, wie dies in US 6,270828, US 6,169,190 oder US 5,965,755 beschrieben ist.
c) Pflanzen wie Rapspflanzen, die Öl mit einem niedrigen gesättigten Fettsäuregehalt produzieren, wie dies z. B. in US 5,434,283 beschrieben ist.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die Herstellung und die Verwendung der erfindungsgemäßen Wirkstoffe soll anhand der folgenden Beispielen näher erläutert werden, ohne jedoch auf diese beschränkt zu sein.

### Herstellungsbeispiele

### N-Ethyl-N-methyl-N'-[4-(3-tertbutylphenoxy)-5-trifluormethyl-2-methylphenyl]formamidin

150 mg (0.46 mmol) 4-(3-tertButylphenoxy)-5-trifluormethyl-2-methylanilin werden in 10 ml Methanol gelöst und mit 0.4 ml einer Lösung von N-Ethyl-N-methylformamiddimethylacetal in Methanol (60%) versetzt. Die Reaktionsmischung wird 12h bei 45°C gerührt, anschliessend im Vakuum vom Lösungsmittel befreit und säulenchromatographisch aufgereinigt. Man erhält 100 mg Produkt (99.9 % Reinheit, 54.9% Ausbeute; log P (pH2.3) = 2.52).

Synthese der Ausgangsverbindungen:

### 4-Brom-3-(difluormethyl)-2-methyl-nitrobenzol

Zu einer Lösung von 9,50 g (28.8 mmol, 74% GC-MS-Reinheit) 2-Brom-4-methyl-5-nitrobenzaldehyd in 150 ml abs. Dichlormethan wurden bei -30°C 12.20 g Diethylaminoschwefeltrifluorid (75.7 mmol) vorsichtig zugetropft. Das Reaktionsgemisch wurde zunächst 2 h bei -30 °C gerührt und anschließend über Nacht auf Raumtemperatur kommen lassen.

Die Reaktionslösung wurde vorsichtig mit 5 ml Methanol gequencht, mit 50 ml Wasser versetzt und zweimal mit Dichlormethan extrahiert. Die vereinigten organischen Phasen wurden getrocknet und am Rotationsverdampfer konzentriert. Der Rückstand wurde über eine Kieselgelkartusche chromatographiert (Cyclohexan/Essigsäureethylester = 10:1).

Man erhielt 8.0 g (22.2 mmol, 74% GC-MS-Reinheit, 77 % der Theorie) an 1-Brom-2-(difluormethyl)-5-methyl-4-nitrobenzol als Feststoff.

### 2-Brom-4-methyl-5-nitrobenzaldehyd

Zu einer Lösung von 11.00 g (55.2 mmol) 2-Brom-4-methylbenzaldehyd in 65 ml Schwefelsäure (95%ig) wurden bei 5°C 11 ml Salpetersäure (68%ig) langsam zugetropft. Die Reaktionstemperatur wurde unter 15°C gehalten. Anschließend wurde die Reaktionsmischung 1 Stunde bei 15°C gerührt und nachfolgend vorsichtig auf Eiswasser gegeben, abfiltriert und mit Wasser gewaschen. Der Feststoff wurde im Hochvakuum getrocknet. Man erhielt man 13.00 g (74% GC-MS-Reinheit, 39.4 mmol, 71% der Theorie) an 2-Brom-4-methyl-5-nitrobenzaldehyd mit dem LogP (HCOOH) = 2,61.

### 4-Chloro-3-trifluormethyl-2-methyl-nitrobenzol

4.9 g (25.2 mmol) 4-Methyl-2-chloro-benzotrifluorid werden in 10 ml konzentrierter Schwefelsäure vorgelegt, auf 0°C abgekühlt und langsam mit einem Gemisch aus 1.5 ml konzentrierter Schwefelsäure und 1.5 ml konzentrierter Salpetersäure versetzt. Man rührt 15 min nach, gibt auf Eis, extrahiert mehrfach mit Dichlormethan, trocknet die vereinten organischen Phasen über MgSO4 und entfernt das Lösungsmittel im Vakuum. Man erhält 4.7 g Produkt (98% Reinheit, 81% Ausbeute, logP(HCOOH): 3.60).

4-Methyl-2-chloro-benzotrifluorid wird entsprechend der Literaturvorschrift hergestellt (J. Fluorine Chem. 1981, 18(3), 281-291).

### 4-(3-tertButylphenoxy)-5-trifluormethyl-2-methylanilin

Eine Lösung von 19.60 g (55.47 mmol) 4-(3-*tert*Butylphenoxy)-5-trifluormethyl-2-methylnitrobenzol in 100 ml Methanol wird zunächst mit 37.55 g (166.41 mmol) Zinn(II)chlorid-Dihydrat bei Raumtemperatur und anschliessend unter Eiskühlung langsam 100 ml konzentrierter Salzsäure versetzt. Es wird 4 h refluxiert, auf Raumtemperatur abgekühlt, eingeengt, mit Wasser versetzt, der Rückstand abfiltriert und im Vakuum getrocknet. Das Rohprodukt wird säulenchromatographisch aufgereinigt. (13.2 g, 86 % Reinheit, 63.5% Ausbeute, log P (pH 2.3) = 4.67).

### 4-(3-tertButylphenoxy)-5-trifluormethyl-2-methylnitrobenzol

3.00 g (19.97 mmol) 3-tertButylphenol, 4.35 g (18.16 mmol) 4-Chlor-5-trifluormethyl-2-methylnitrobenzol und 3.26 g (23.60 mmol) Kaliumcarbonat werden in 50 ml N,N-Dimethylformamid 15 min auf 50°C erhitzt refluxiert. Man gibt die Reaktionslösung auf Wasser, extrahiert mehrfach mit Dichlormethan, trocknet über Na₂SO₄, filtriert und entfernt das Lösungsmittel im Vakuum. Der Feststoff wird mit Wasser und Hexan gewaschen und im Vakuum getrocknet. (6.6 g, 70.0 % Reinheit, 72.0 % Ausbeute, log P (pH 2.3) = 5.68).

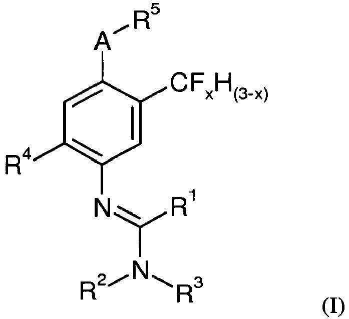

**Tabelle I**

| **Nr.** | **logP neutral** | **logP sauer** | **A** | **R¹** | **R²** | **R³** | **R⁴** | **x** | **R⁵** |
|---|---|---|---|---|---|---|---|---|---|
| 1 | | 2,93 | O | H | Et | Me | Me | 1 | 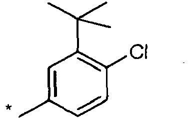 |
| 2 | | 2,67 | bond | H | Et | Me | Me | 1 | 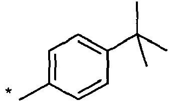 |
| 3 | 6,68 | 3,26 | O | H | Et | Me | Me | 3 | 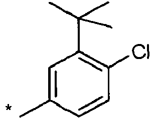 |
| 4 | 5,74 | 2,65 | O | H | Et | Me | Me | 3 | 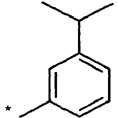 |
| 5 | 5,77 | 2,81 | O | H | Et | Me | Me | 3 | 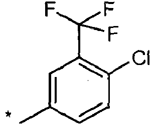 |
| 6 | 6,15 | 3,01 | O | H | Pr | Me | Me | 3 | 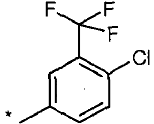 |
| | | | | | | | | | |
| 8 | 6,30 | 3,13 | O | H | Me | Me | Me | 3 | 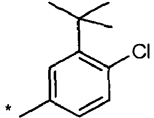 |
| 9 | | 2,82 | O | H | Et | Me | Me | 2 | 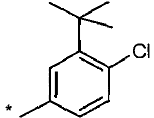 |
| 10 | | 2,31 | O | H | Et | Me | Me | 2 | 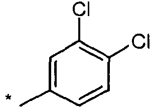 |
| 11 | 6,04 | 2,52 | O | H | Et | Me | Me | 3 | 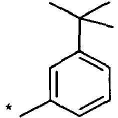 |
| 12 | 6,15 | 2,74 | bond | H | Et | Me | Me | 3 | 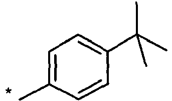 |
| 13 | | 1,73 | O | H | Et | Me | Me | 2 | 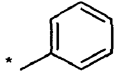 |
| | | | | | | | | | |
| 15 | | 1,65 | O | H | Et | Me | Me | 2 | 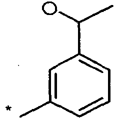 |
| 16 | | 2,17 | O | H | Et | Me | Me | 2 | 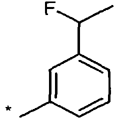 |
| 17 | | 2,97 | O | H | Et | Me | Me | 2 | 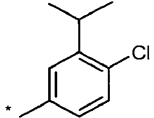 |
| 18 | | 2,50 | O | H | Et | Me | Me | 2 | 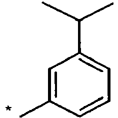 |
| | | | | | | | | | |
| | | | | | | | | | |
| | | | | | | | | | |
| | | | | | | | | | |
| | | | | | | | | | |
| | | | | | | | | | |

### Verwendungsbeispiele

### Alternaria-Test (Tomate) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Alternaria solani*** inokuliert. Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **3, 5, 9, 12** und **13** (siehe Tab. I) bei einer Konzentration an Wirkstoff von 100 ppm einen Wirkungsgrad von 70% oder mehr.

### Uromyces - Test (Bohne) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Bohnenrosterregers ***Uromyces appendiculatus*** inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **3, 4, 5, 9, 10, 12** und **13** (siehe Tab. I) bei einer Konzentration an Wirkstoff von 100 ppm einen Wirkungsgrad von 70% oder mehr.

### Sphaerotheca-Test (Gurke) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Sphaerotheca fuliginea* inokuliert. Anschließend werden die Pflanzen in einem Gewächshaus bei 70% relativer Luftfeuchtigkeit und einer Temperatur von 23 C aufgestellt.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **3, 4, 5, 6, 8, 9, 10, 12, 13, 15** und **16** (siehe Tab. I) bei einer Konzentration an Wirkstoff von 500 ppm einen Wirkungsgrad von 70% oder mehr.

### Erysiphe-Test (Gerste) / protektiv

| | |
|---|---|
| Lösungsmittel: | 49 Gewichtsteile N,N-Dimethylacetamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit, besprüht man junge Pflanzen mit der Wirkstoffzubereitung in der angegebenen Aufwandmenge. Nach Antrocknen des Spritzbelages werden die Pflanzen mit Sporen von *Erysiphe graminis f.sp. hordei* bestäubt. Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **1, 2, 3, 4, 6, 8, 9, 10** und **12** bei einer Konzentration an Wirkstoff von 1000 ppm einen Wirkungsgrad von 70% oder mehr.

### Puccinia-Test (Weizen) / protektiv

| | |
|---|---|
| Lösungsmittel: | 49 Gewichtsteile N,N-Dimethylacetamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von *Puccinia recondita* besprüht. Die Pflanzen verbleiben 48 Stunden bei 20°C und 100% relativer Luftfeuchtigkeit in einer Inkubationskabine. Die Pflanzen werden dann in einem Gewächshaus bei einer Temperatur von ca. 20°C und einer relativen Luftfeuchtigkeit von 80% aufgestellt, um die Entwicklung von Rostpusteln zu begünstigen.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **1, 2, 3, 4, 6, 8, 9, 10** und **12** bei einer Konzentration an Wirkstoff von 1000 ppm einen Wirkungsgrad von 70% oder mehr.

## Patentansprüche

1. Fluoralkylphenylamidine der Formel (I) 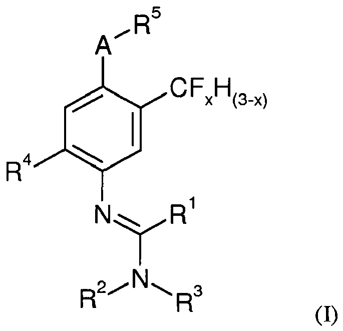 in welcher
x eine ganze Zahl, ausgewählt aus 1, 2 und 3 ist;
A ist O;
R¹ ausgewählt ist aus Wasserstoff; linearen oder verzweigten C₁₋₁₂-Alkyl-, C₂₋₁₂-Alkenyl-, C₂₋₁₂-Alkinyl-Gruppen, cyclischen C₃₋₁₂-Alkyl-, C₄₋₁₂-Alkenyl- oder C₄₋₁₂-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' Wasserstoff oder eine C₁₋₁₂-Alkylgruppe sein kann;
X ein Halogenatom ist, das ausgewählt ist aus Fluor, Chlor, Brom und Iod;
-SH; -SR", wobei R" eine lineare oder verzweigte C₁₋₁₂-Alkylgruppe sein kann, die substituiert sein kann mit einer oder mehreren Gruppen, die ausgewählt sind aus - R', -X, -OR', -SR', -NR'₂, -SiR'₃, -COOR', -CN und-CONR₂', worin R' die obigen Bedeutungen hat;
R² ausgewählt ist aus linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -NR'₂, -SiR'₃, -COOR', -CN und -CONR₂', substituiert sein können, wobei R' die obigen Bedeutungen hat;
R³ ausgewählt ist aus -CN, -SH, -SR", -OR", -(C=O)-R", linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -NR'₂, -SiR'₃, -COOR', -CN und -CONR₂', substituiert sein können, wobei R' und R" die obigen Bedeutungen haben;
R⁴ ausgewählt ist aus der Gruppe bestehend aus -X, -CN, -SH, -SR", -OR", -(C=O)-R", linearen oder verzweigten C₁₋₁₂-Alkyl-, C₂₋₁₂-Alkenyl-, C₂₋₁₂-Alkinyl-Gruppen, cyclischen C₃₋₈-Alkyl-, C₄₋₈-Alkenyl-, C₄₋₈-Alkinyl-Gruppen, C₅₋₁₈-Aryl-, C₇₋₁₉-Ar-alkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -NR'₂, -SiR'₃, -COOR', -CN und -CONR₂', substituiert sein können, wobei R' und R" die obigen Bedeutungen haben;
R⁵ ist 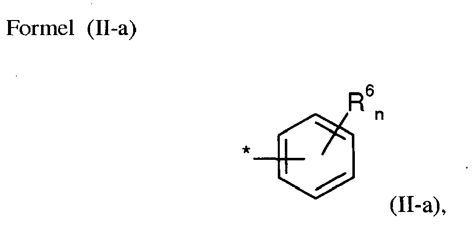 in der
n eine ganze Zahl von 0, 1, 2 oder 3 ist;
R⁶ ausgewählt ist aus Wasserstoff, linearen C₁₋₁₂-Alkyl-Gruppen, verzweigten oder cyclischen C₃₋₈-Alkyl-Gruppen, Halogenatomen, C₁₋₅-Haloalkyl-Gruppen
und deren Salze.

2. Fluoralkylphenylamidine gemäß Anspruch 1, wobei
A ist O;
x eine ganze Zahl, ausgewählt aus 2 oder 3 ist;
R¹ ausgewählt ist aus der Gruppe bestehend aus Wasserstoff, Mercapto (-SH) oder C₁₋₈-Alkyl-Gruppen;
R² ausgewählt ist aus C₁₋₈-Alkyl-Gruppen;
R³ ausgewählt ist aus linearen C₂₋₈-Alkyl-Gruppen, verzweigten und alicyclischen C₃₋₈-Alkyl-Gruppen;
und
R⁴ ausgewählt ist aus der Gruppe bestehend aus -X, linearen oder verzweigten, C₁₋₈-Alkyl-Gruppen und C₁₋₅-Haloalkyl-Gruppen.

3. Fluoralkylphenylamidine gemäß einem der Ansprüche 1 oder 2, wobei
A ist O;
x eine ganze Zahl, ausgewählt aus 1, 2 oder 3 ist;
R¹ ausgewählt ist aus der Gruppe bestehend aus Wasserstoff, Methyl und Ethyl;
R² ausgewählt ist aus der Gruppe bestehend aus Methyl und Ethyl;
R³ ausgewählt ist aus der Gruppe bestehend aus Methyl, Ethyl und Cyclopropyl;
R⁴ ausgewählt sein kann aus Cl- und F-Atomen und -CF₃, -CF₂H, -CH₂F und MethylGruppen;
und R⁵ ausgewählt ist aus den Gruppen (i) bis (iii)
(i) Gruppen der Formel (II-g) 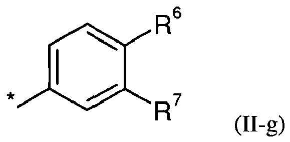 in welcher
R⁶ Halogen, eine C₁₋₈-Alkyl-Gruppe, eine C₁₋₅-Halogenalkyl-Gruppe ist und
R⁷ Wasserstoff, Halogen, Chlor oder Fluor, eine C₁₋₈-Alkyl-Gruppe oder eine C₁₋₅-HalogenaLkyl-Gruppe ist,
oder R⁶ und R⁷ zusammen mit den Ringatomen, an die sie gebunden sind oder mit weiteren Atomen, ausgewählt aus N, O, P und S, einen vier- bis siebengliedrigen Ring bilden können, der mit einer oder mehreren X-, R'-, OR'-, SR'-, NR'₂-, SiR'₃-Gruppen substituiert sein kann, wobei R' die obigen Bedeutungen hat;
X ein Halogenatom ist, das ausgewählt ist aus Fluor, Chlor, Brom und Iod;
(ii) Gruppen der Formel (II-h) 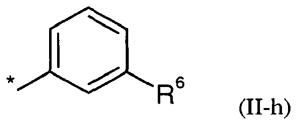 in welcher
R⁶ Halogen, eine lineare oder verzweigte C₁₋₈-Alkyl-Gruppe, eine C₃₋₁₂ Cycloalkyl-, C₅₋₁₈-Aryl-, C₇₋₁₉-Aralkyl- oder C₇₋1₉-Alkaryl-Gruppen sein kann, wobei die zuvor genannten Gruppen mit SiR'₃-, -OR' und -CN substituiert sein können, wobei R' die obigen Bedeutungen hat ist.

4. Fluoralkylphenylamidine, gemäß einem der Ansprüche 1 bis 3, ausgewählt aus der Gruppe bestehend aus (1) N-Ethyl-N-methyl-N'-[4-(3-*tert*-butyl-4-chlorophenoxy)-5-fluormethyl-2-methylphenyl]formamidine, (2) N-Ethyl-N-methyl-N'-[4-(4-*tert*-butyl-phenyl)-5-fluormethyl-2-methylphenyl]formamidine, (3) N-Ethyl-N-methyl-N'-[4-(3-*tert*-butyl-4-chlorophenoxy)-5-trifluormethyl-2-methylphenyl]formamidine, (4) N-Ethyl-N-methyl-N'-[4-(3-*iso*-propyl-phenoxy)-5-trifluormethyl-2-methylphenyl]formamidine, (5) N-Ethyl-N-methyl-N'-[4-(3-trifluormethyl-4-chlorophenoxy)-5-trifluormethyl-2-methylphenyl]formamidine, (6) N-Propyl-N-methyl-N'-[4-(3-trifluormethyl-4-chlorophenoxy)-5-trifluormethyl-2-methylphenyl]formamidine, (8) N,N-Dimethyl-N'-[4-(3-*tert*-butyl-4-chlorophenoxy)-5-trifluormethyl-2-methylphenyl]-formamidine, (9) N-Ethyl-N-methyl-N'-[4-(3-*tert*-butyl-4-chlorophenoxy)-5-difluormethyl-2-methylphenyl]formamidine (10) N-Ethyl-N-methyl-N'-[4-(3,4-dichlorophenoxy)-5-difluormethyl-2-methylphenyl]formamidine, (11) N-Ethyl-N-methyl-N'-[4-(3-*tert*-butyl-phenoxy)-5-trifluormethyl-2-methylphenyl]formamidine, (12) N-Ethyl-N-methyl-N'-[4-(4-*tert*-butyl-phenyl)-5-trifluormethyl-2-ethylphenyl]formamidine, (13) N-Ethyl-N-methyl-N'-[4-phenoxy-5-difluormethyl-2-methylphenyl]formamidine, (15) N-Ethyl-N-methyl-N'-{4-[3-(1-hydroxyethyl)-phenoxy]-5-difluormethyl-2-methylphenyl}formamidine, (16) N-Ethyl-N-methyl-N'-{4-[3-(1-fluorethyl)-phenoxy]-5-difluormethyl-2-methylphenyl}formamidine, (17) N-Ethyl-N-methyl-N'-[4-(3-iso-propyl-4-chlorophenoxy)-5-difluormethyl-2-methylphenyl]formamidine, (18) N-Ethyl-N-methyl-N'-[4-(3-*iso*-propyl-phenoxy)-5-difluormethyl-2-methylphenyl]formamidine.

5. Verfahren zum Herstellen der Fluoralkylphenylamidine gemäß einem der Ansprüch 1 bis 4 umfassend wenigstens einen der folgenden Schritte (a) bis (n):
(a) Umsetzung von Nitrobenzolderivaten der Formel (III) mit Alkoholen oder Phenolen der Formel (II) gemäß dem nachfolgenden Reaktionsschema: 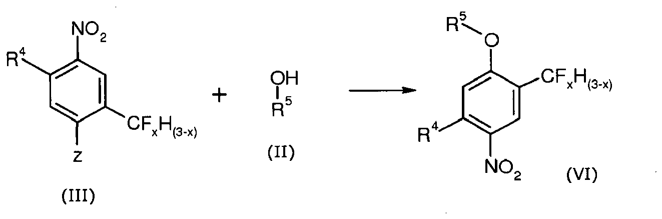
(b) Umsetzung von Nitrophenolderivaten der Formel (V) mit Phenylderivaten der Formel (IV) gemäß dem nachfolgenden Reaktionsschema: 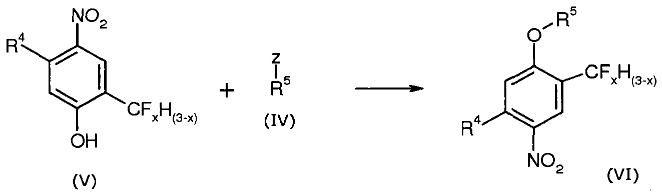
(c) Umsetzung von Anilinen der Formel (VII) mit Alkoholen oder Phenolen der Formel (II) gemäß dem nachfolgenden Reaktionsschema: 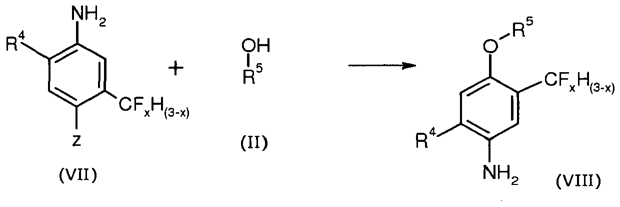
(d) Umsetzung von Aminophenolen der Formel (XII) mit Phenylderivaten der Formel (IV) gemäß dem nachfolgenden Reaktionsschema: 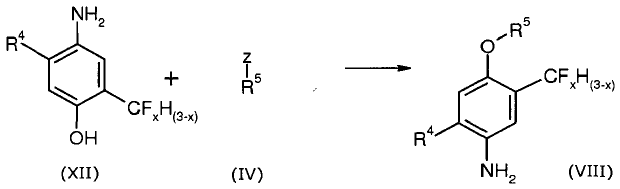
(e) Reduktion der Fluoralkylnitrophenole der Formel (VI) zu Fluoralkylanilinen der Formel (VIII) gemäß dem nachfolgenden Reaktionsschema: 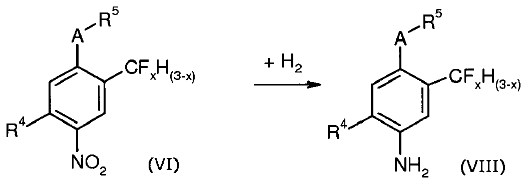
(f) Umsetzung der Anilinether der Formel (VIII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI) gemäß dem nachfolgenden Reaktionsschema: 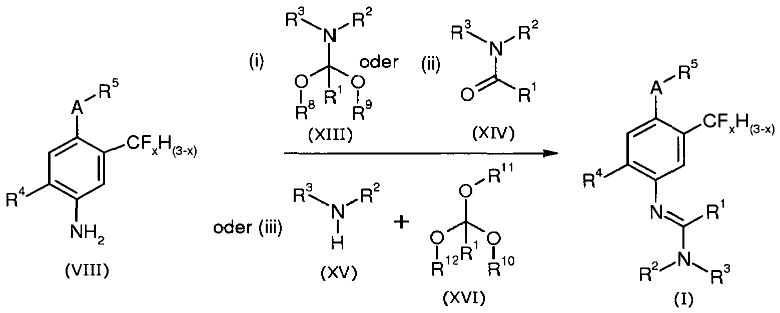
(g) Umsetzung der Aminophenole der Formel (XII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 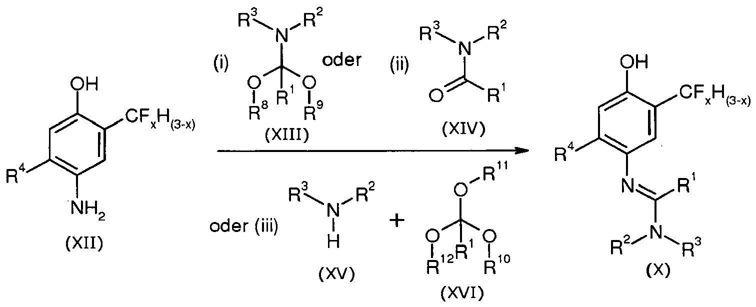
(h) Umsetzung der Aminophenole der Formel (VII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)gemäß dem nachfolgenden Reaktionsschema: 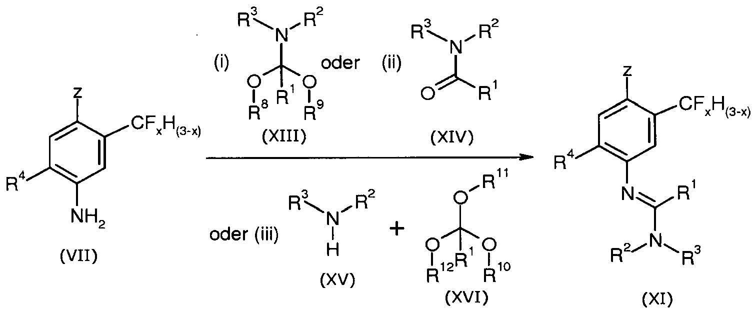
(i) Umsetzung von Amidinen der Formel (XI) mit Alkoholen oder Phenolen der Formel (II) gemäß dem nachfolgenden Reaktionsschema: 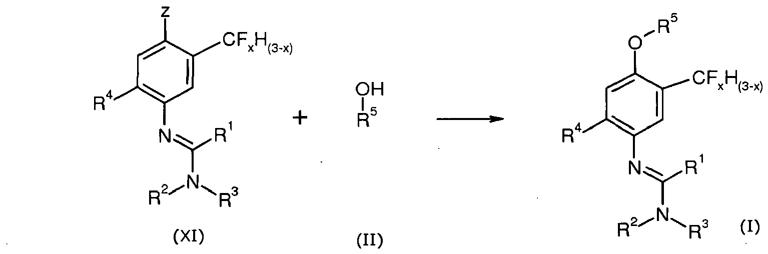
(j) Umsetzung von Amidinen der Formel (XI) mit Phenylderivaten der Formel (IV) gemäß dem nachfolgenden Reaktionsschema: 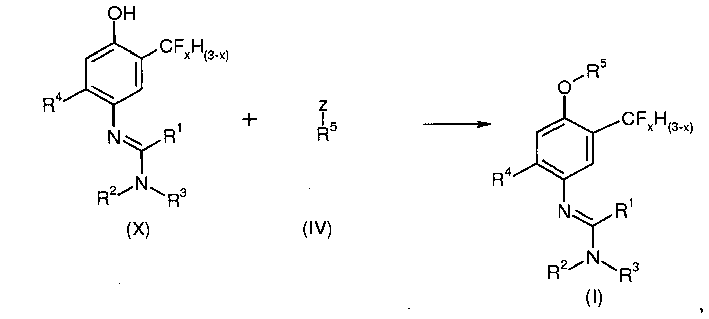
(k) Umsetzung von Fluoralkylanilinen der Formel (VII) mit Boronsäurederivaten der Formel (XVII) gemäß dem nachfolgenden Reaktionsschema: 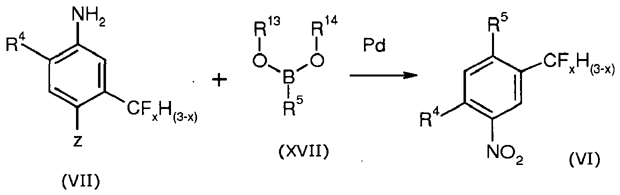 wobei in den zuvor beschriebenen Formeln
x, Z, R¹ bis R⁵ die obigen Bedeutungen haben;
R¹³ und R¹⁴ unabhängig voneinander ausgewählt sind aus der Gruppe bestehend aus Wasserstoff, C₁₋₁₂-Alkyl-, C₂₋₁₂-Alkenyl- C₂₋₁₂-Alkinyl- oder C₅₋₁₈-Aryl- oder C₇₋₁₉-Arylalkyl-Gruppen und die gemeinsam mit den O-Atomen, an die sie gebunden sind, einen fünf-, sechs- oder siebengliedrigen Ring bilden können.
(l) Umsetzung von Fluoralkylanilinen der Formel (VII) mit Aldehyden oder Ketonen der Formel (XVIII) gemäß dem nachfolgenden Reaktionsschema: 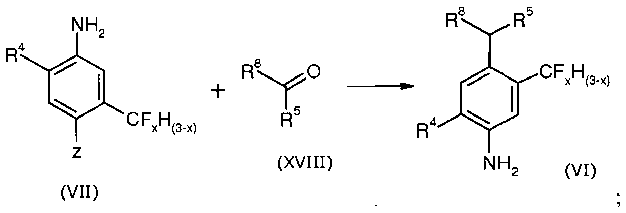
(m) Umsetzung von Fluoralkylnitrobenzolen der Formel (III) mit Boronsäurederivaten der Formel (XVII) gemäß dem nachfolgenden Reaktionsschema: 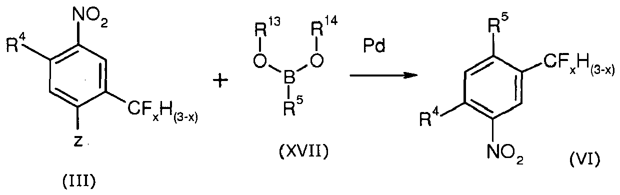 wobei in den zuvor beschriebenen Formeln
(n) Umsetzung von Fluoralkylnitrobenzolen der Formel (III) mit Aldehyden oder Ketonen der Formel (XVIII) gemäß dem nachfolgenden Reaktionsschema: 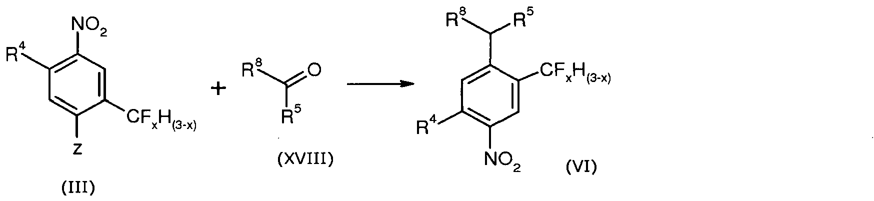 wobei in den zuvor beschriebenen Formeln
x, Z, R¹ bis R⁸ die obigen Bedeutungen haben;
R¹³ und R¹⁴ unabhängig voneinander ausgewählt sind aus der Gruppe bestehend aus Wasserstoff, C₁₋₁₂-Alkyl-, C₂₋₁₂-Alkenyl- C₂₋₁₂-Alkinyl- oder C₅₋₁₈-Aryl- oder C₇₋₁₉-Arylalkyl-Gruppen und die gemeinsam mit den O-Atomen, an die sie gebunden sind, einen fünf-, sechs- oder siebengliedrigen Ring bilden können.

6. Mittel zum Bekämpfen unerwünschter Mikroorganismen, umfassend wenigstens ein Fluoralkylphenylamidin gemäß einem der Ansprüche 1 bis 4.

7. Nicht therapeutische Verwendung eines Fluoralkylphenylamidins gemäß irgendeinem der Ansprüche 1 bis 4 oder Mischungen dieser zum Bekämpfen unerwünschter Mikroorganismen.

8. Nicht therapeutisches Verfahren zum Bekämpfen unerwünschter Mikroorganismen, **dadurch gekennzeichnet, dass** Fluoralkylphenylamidine gemäß einem der Ansprüche 1 bis 4 auf die Mikroorganismen und/oder in deren Lebensraum ausgebracht werden.

9. Saatgut, welches mit wenigstens einem Fluoralkylphenylamidin gemäß einem der Ansprüche 1 bis 4 behandelt ist.

10. Verwendung von Fluoralkylphenylamidinen gemäß einem der Ansprüche 1 bis 4 zur Behandlung von Saatgut.

11. Verwendung von Fluoralkylphenylamidinen gemäß einem der Ansprüche 1 bis 4 zur Behandlung von transgenen Pflanzen.

12. Verwendung von Fluoralkylphenylamidinen gemäß einem der Ansprüche 1 bis 4 zur Behandlung von Saatgut transgener Pflanzen.

13. Verfahren zum Schutz von Saatgut vor unerwünschten Mikroorganismen durch Verwendung eines mit wenigstens einem Fluoralkylphenylamidin gemäß einem der Ansprüche 1 bis 4 behandelten Saatgut.

## Claims

1. Fluoroalkylphenylamidines of the formula (I) 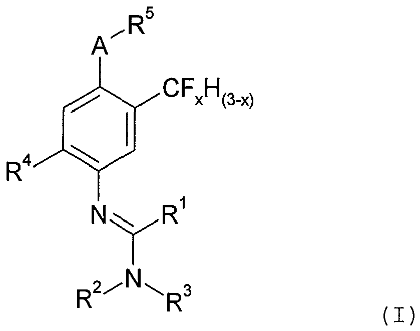 in which
x represents an integer selected from the group consisting of 1, 2 and 3;
A represents O;
R¹ is selected from the group consisting of hydrogen; straight-chain or branched C₁₋₁₂-alkyl, C₂₋₁₂-alkenyl, C₂₋₁₂-alkynyl groups, cyclic C₃₋₁₂-alkyl, C₄₋₁₂-alkenyl or C₄₋₁₂-alkynyl groups, where in the ring system of all of the cyclic groups mentioned above one or more carbon atoms may be replaced by heteroatoms selected from the group consisting of N, O, P and S and all of the groups mentioned above may be substituted by one or more groups selected from the group consisting of -R', -X, -OR', -SR', -NR'₂, -SiR'₃, -COOR', -CN and -CONR'₂, where R' may represent hydrogen or a C₁₋₁₂-alkyl group;
X represents a halogen atom which is selected from the group consisting of fluorine, chlorine, bromine and iodine;
-SH; -SR", where R" may represent a straight-chain or branched C₁₋₁₂-alkyl group which may be substituted by one or more groups selected from the group consisting of -R', -X, -OR', -SR', -NR'₂, -SiR'₃, -COOR', -CN and -CONR'₂, where R' has the above meanings;
R² is selected from the group consisting of straight-chain or branched C₁₋₁₂-alkyl, C₂₋₁₂-alkenyl, C₂₋₁₂-alkynyl groups, cyclic C₃₋₈-alkyl, C₄₋₈-alkenyl, C₄₋₈-alkynyl groups, C₅₋₁₈-aryl, C₇₋₁₉-aralkyl and C₇₋₁₉-alkaryl groups, where in the ring system of all of the cyclic groups mentioned above one or more carbon atoms may be replaced by heteroatoms selected from the group consisting of N, O, P and S and all of the groups mentioned above may be substituted by one or more groups selected from the group consisting of -R', -X, -OR', -SR', -NR'₂, -SiR'₃, -COOR', -CN and -CONR'₂, where R' has the above meanings;
R³ is selected from the group consisting of -CN, -SH, -SR" , -OR", -(C=O)-R", straight-chain or branched C₁₋₁₂-alkyl, C₂₋₁₂-alkenyl, C₂₋₁₂-alkynyl groups, cyclic C₃₋₈-alkyl, C₄₋₈-alkenyl, C₄₋₈-alkynyl groups, C₅₋₁₈-aryl, C₇₋₁₉-aralkyl and C₇₋₁₉-alkaryl groups, where in the ring system of all of the cyclic groups mentioned above one or more carbon atoms may be replaced by heteroatoms selected from the group consisting of N, O, P and S and all of the groups mentioned above may be substituted by one or more groups selected from the group consisting of -R', -X, -OR', -SR', -NR'₂, -SiR'₃, -COOR', -CN and -CONR'₂, where R' and R" have the above meanings;
R⁴ is selected from the group consisting of -X, - CN, -SH, -SR", -OR", - (C=O)-R", straight-chain or branched C₁₋₁₂-alkyl, C₁₋₁₂-alkenyl, C₂₋₁₂-alkynyl groups, cyclic C₃₋₈-alkyl, C₄₋₈-alkenyl, C₄₋₈-alkynyl groups, C₅₋₁₈-aryl, C₇₋₁₉-aralkyl and C₇₋₁₉-alkaryl groups, where in the ring system of all of the cyclic groups mentioned above one or more carbon atoms may be replaced by heteroatoms selected from the group consisting of N, O, P and S and all of the groups mentioned above may be substituted by one or more groups selected from the group consisting of -R', -X, -OR', -SR', -NR'₂, -SiR'₃, -COOR', -CN and -CONR'₂, where R' and R" have the above meanings;
R⁵ represents 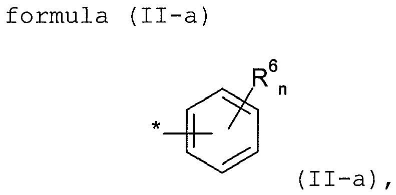 in which
n represents an integer of 0, 1, 2 or 3;
R⁶ is selected from the group consisting of hydrogen, straight-chain C₁₋₁₂-alkyl groups, branched or cyclic C₃₋₈-alkyl groups, halogen atoms, C₁₋₅-haloalkyl groups
and salts thereof.

2. Fluoroalkylphenylamidines according to Claim 1 where
A represents O;
x represents an integer selected from the group consisting of 2 and 3;
R¹ is selected from the group consisting of hydrogen, mercapto (-SH) or C₁₋₈-alkyl groups;
R² is selected from the group consisting of C₁₋₈-alkyl groups;
R³ is selected from the group consisting of straight-chain C₂-C₈-alkyl groups, branched and alicyclic C₃₋₈-alkyl groups;
and
R⁴ is selected from the group consisting of -X, straight-chain or branched C₁₋₈-alkyl groups and C₁₋₅-haloalkyl groups.

3. Fluoroalkylphenylamidines according to Claim 1 or 2 where
A represents O;
x represents an integer selected from the group consisting of 1, 2 and 3;
R¹ is selected from the group consisting of hydrogen, methyl and ethyl;
R² is selected from the group consisting of methyl
and ethyl;
R³ is selected from the group consisting of methyl, ethyl and cyclopropyl;
R⁴ may be selected from the group consisting of Cl and F atoms and -CF₃, -CF₂H, -CH₂F and methyl groups;
and R⁵ is selected from groups (i) to (ii)
(i) groups of the formula (II-g) 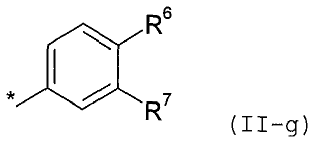 in which
R⁶ represents halogen, a C₁₋₈-alkyl group, a C₁₋₅-haloalkyl group and
R⁷ represents hydrogen, halogen, chlorine or fluorine, a C1-8-alkyl group or a C1-5-haloalkyl group,
or R⁶ and R⁷ together with the ring atoms to which they are attached or together with further atoms selected from the group consisting of N, O, P and S may form a four- to seven-membered ring which may be substituted by one or more X, R', OR', SR', NR'₂, SiR'₃ groups, where R' has the above meanings;
X represents a halogen atom which is selected from the group consisting of fluorine, chlorine, bromine and iodine;
(ii) groups of the formula (II-h) 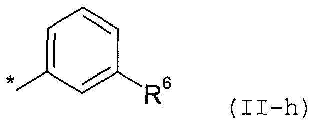 in which
R⁶ may represent halogen, a straight-chain or branched C₁₋₈-alkyl group, C₃₋₁₂-cycloalkyl, C₅-₁₈-aryl, C₇₋₁₉-aralkyl and C₇₋₁₉-alkaryl groups, where the groups mentioned above may be substituted with -SiR'₃, -OR' and -CN, where R' has the above meanings.

4. Fluoroalkylphenylamidines according to any of Claims 1 to 3 selected from the group consisting of (1) N-ethyl-N-methyl-N'-[4-(3-tert-butyl-4-chlorophenoxy)-5-fluoromethyl-2-methylphenyl]formamidine, (2) N-ethyl-N-methyl-N'-[4-(4-*tert*-butylphenyl)-5-fluoromethyl-2-methylphenyl]formamidine, (3) N-ethyl-N-methyl-N'-[4-(3-*tert*-butyl-4-chlorophenoxy)-5-trifluoromethyl-2-methylphenyl]formamidine, (4) N-ethyl-N-methyl-N'-[4-(3-iso-propylphenoxy)-5-trifluoromethyl-2-methylphenyl]formamidine, (5) N-ethyl-N-methyl-N'-[4-(3-trifluoromethyl-4-chlorophenoxy)-5-trifluoromethyl-2-methylphenyl]-formamidine, (6) N-propyl-N-methyl-N'-[4-(3-trifluoromethyl-4-chlorophenoxy)-5-trifluoromethyl-2-methylphenyl]formamidine, (8) N,N-dimethyl-N'-[4-(3-*tert*-butyl-4-chlorophenoxy)-5-trifluoromethyl-2-methylphenyl]formamidine, (9) N-ethyl-N-methyl-N'-[4-(3-*tert*-butyl-4-chlorophenoxy)-5-difluoromethyl-2-methylphenyl]formamidine, (10) N-ethyl-N-methyl-N'-[4-(3,4-dichlorophenoxy)-5-difluoromethyl-2-methylphenyl]formamidine, (11) N-ethyl-N-methyl-N'-[4-(3-*tert*-butylphenoxy)-5-trifluoromethyl-2-methylphenyl]formamidine, (12) N-ethyl-N-methyl-N'-[4-(4-*tert*-butylphenyl)-5-trifluoromethyl-2-ethylphenyl]formamidine, (13) N-ethyl-N-methyl-N'-[4-phenoxy-5-difluoromethyl-2-methylphenyl]formamidine, (15) N-ethyl-N-methyl-N'-{4-[3-(1-hydroxyethyl)-phenoxy]-5-difluoromethyl-2-methylphenyl}formamidine, (16) N-ethyl-N-methyl-N'-{4-[3-(1-fluoroethyl)-phenoxy]-5-difluoromethyl-2-methylphenyl}formamidine, (17) N-ethyl-N-methyl-N'-[4-(3-isopropyl-4-chlorophenoxy)-5-difluoromethyl-2-methylphenyl]formamidine, (18) N-ethyl-N-methyl-N'-[4-(3-iso-propylphenoxy)-5-difluoromethyl-2-methylphenyl]formamidine.

5. Process for preparing the fluoroalkylphenylamidines according to any of Claims 1 to 4 which comprises at least one of the following steps (a) to (n):
(a) reaction of nitrobenzene derivatives of the formula (III) with alcohols or phenols of the formula (II) according to the reaction scheme below: 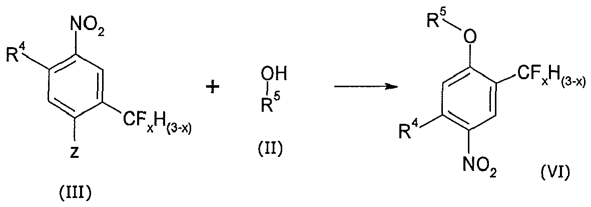
(b) reaction of nitrophenol derivatives of the formula (V) with phenyl derivatives of the formula (IV) according to the reaction scheme below: 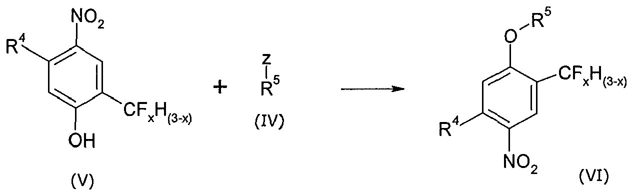
(c) reaction of anilines of the formula (VII) with alcohols or phenols of the formula (II) according to the reaction scheme below: 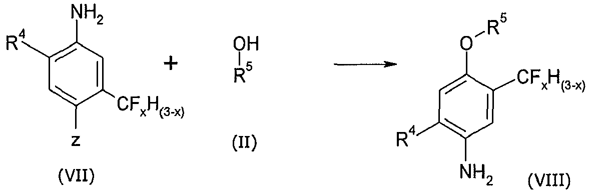
(d) reaction of aminophenols of the formula (XII) with phenyl derivatives of the formula (IV) according to the reaction scheme below: 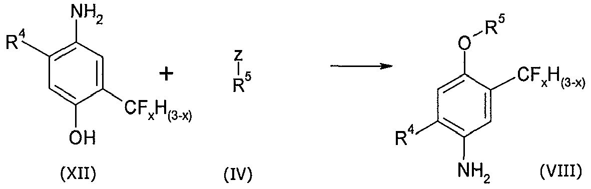
(e) reduction of the fluoroalkylnitrophenols of the formula (VI) to fluoroalkylanilines of the formula (VIII) according to the reaction scheme below: 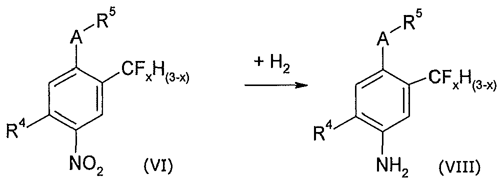
(f) reaction of the aniline ethers of the formula (VIII) with
(i) aminoacetals of the formula (XIII) or
(ii) with amides of the formula (XIV) or
(iii)with amines of the formula (XV) in the presence of ortho esters of the formula (XVI) according to the reaction scheme below: 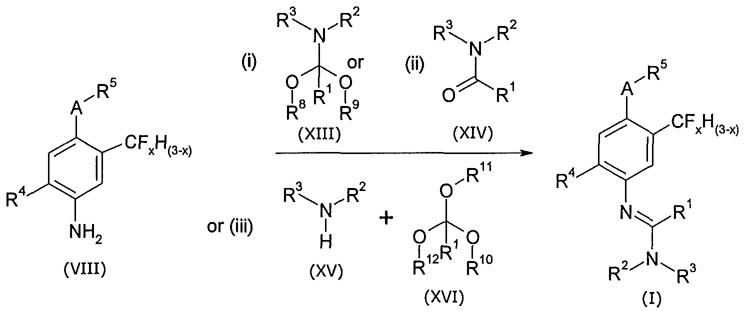
(g) reaction of the aminophenols of the formula (XII) with
(i) aminoacetals of the formula (XIII) or
(ii) with amides of the formula (XIV) or
(iii)with amines of the formula (XV) in the presence of ortho esters of the formula (XVI)
according to the reaction scheme below: 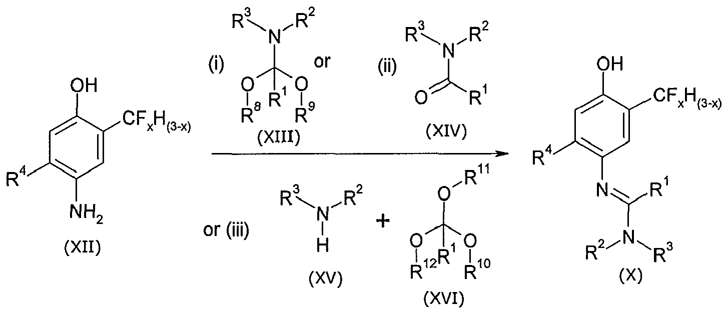
(h) reaction of the aminophenols of the formula (VII) with
(i) aminoacetals of the formula (XIII) or
(ii) with amides of the formula (XIV) or
(iii)with amines of the formula (XV) in the presence of ortho esters of the formula (XVI) according to the reaction scheme below: 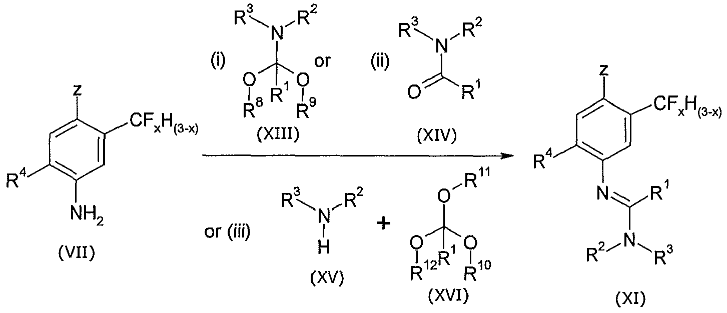
(i) reaction of amidines of the formula (XI) with alcohols or phenols of the formula (II) according to the reaction scheme below: 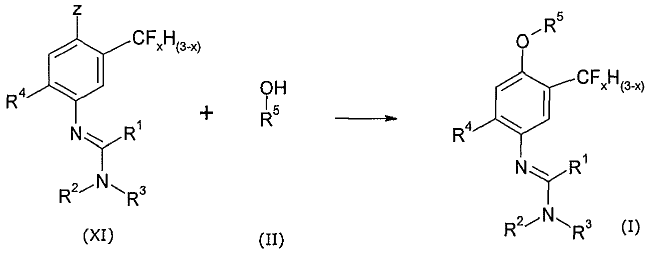
(j) reaction of amidines of the formula (XI) with phenyl derivatives of the formula (IV) according to the reaction scheme below: 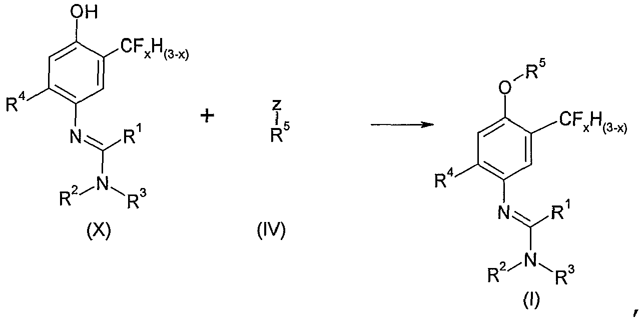
(k) reaction of fluoroalkylanilines of the formula (VII) with boronic acid derivatives of the formula (XVII) according to the reaction scheme below: 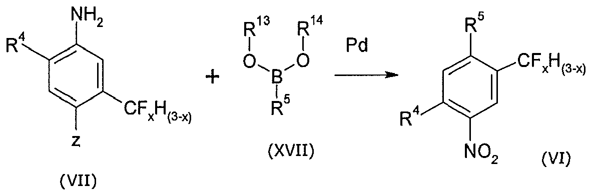 where in the formulae described above
x, Z, R¹ to R⁵ have the above meanings;
R¹³ and R¹⁴ independently of one another are selected from the group consisting of hydrogen, C₁₋₁₂-alkyl, C₂₋₁₂-alkenyl, C₂₋₁₂-alkynyl or C₅₋₁₈-aryl or C₇₋₁₉-arylalkyl groups and which together with the oxygen atoms to which they are attached may form a five-, six- or seven-membered ring,
(l) reaction of fluoroalkylanilines of the formula (VII) with aldehydes or ketones of the formula (XVIII) according to the reaction scheme below: 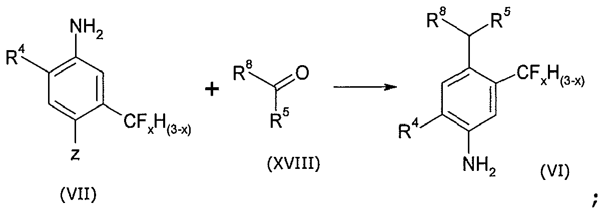
(m) reaction of fluoroalkylnitrobenzenes of the formula (III) with boronic acid derivatives of the formula (XVII) according to the reaction scheme below: 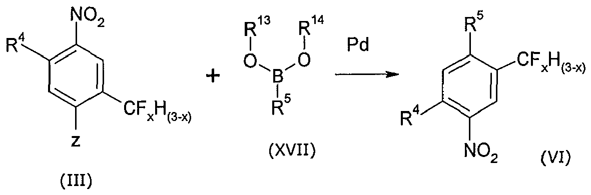
(n) reaction of fluoroalkylnitrobenzenes of the formula (III) with aldehydes or ketones of the formula (XVIII) according to the reaction scheme below: 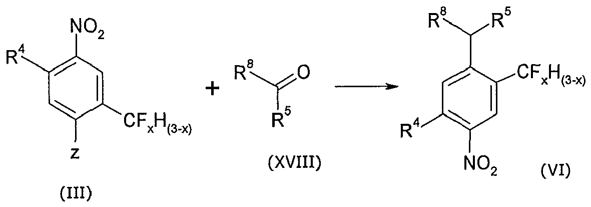
x, Z, R¹ to R⁸ have the above meanings;
R¹³ and R¹⁴ independently of one another are selected from the group consisting of hydrogen, C₁₋₁₂-alkyl, C₂₋₁₂-alkenyl, C₂₋₁₂-alkynyl or C₅₋₁₈-aryl or C₇₋₁₉-arylalkyl groups and which together with the oxygen atoms to which they are attached may form a five-, six- or seven-membered ring.

6. Composition for controlling unwanted microorganisms, comprising at least one fluoroalkylphenylamidine according to any of Claims 1 to 4.

7. Nontherapeutic use of a fluoroalkylphenylamidine according to any of Claims 1 to 4 or of mixtures of these for controlling unwanted microorganisms.

8. Nontherapeutic method for controlling unwanted microorganisms, **characterized in that** fluoroalkylphenylamidines according to any of Claims 1 to 4 are applied to the microorganisms and/or their habitat.

9. Seed treated with at least one fluoroalkylphenylamidine according to any of Claims 1 to 4.

10. Use of fluoroalkylphenylamidines according to any of Claims 1 to 4 for treating seed.

11. Use of fluoroalkylphenylamidines according to any of Claims 1 to 4 for treating transgenic plants.

12. Use of fluoroalkylphenylamidines according to any of Claims 1 to 4 for treating seed of transgenic plants.

13. Method for protecting seed against unwanted microorganisms by using seed treated with at least one fluoroalkylphenylamidine according to any of Claims 1 to 4.

## Revendications

1. Fluoroalkylphénylamidines de formule (I) 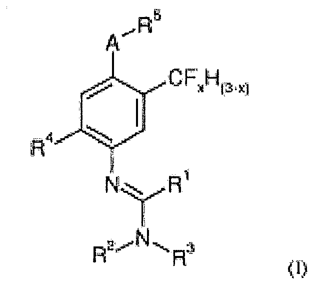 dans laquelle
x est un nombre entier, choisi parmi 1, 2 et 3 ;
A est O ;
R¹ est choisi parmi un atome d'hydrogène ; des groupes alkyle en C₁-C₁₂, alcényle en C₂-C₁₂, alcynyle en C₂-C₁₂ linéaires ou ramifiés, des groupes alkyle en C₃-C₁₂, alcényle en C₄-C₁₂ ou alcynyle en C₄-C₁₂ cycliques, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR'₂, R' étant un atome d'hydrogène ou un groupe alkyle en C₁-C₁₂ ;
X étant un atome d'halogène qui est choisi parmi le fluor, le chlore, le brome et l'iode ;
-SH ; -SR", R" pouvant être un groupe alkyle en C₁-C₁₂ linéaire ou ramifié qui peut être substitué par un ou plusieurs groupes qui sont choisis parmi -R', -X, -OR', -SR', -NR'₂, -SiR'₃, -COOR', -CN et -CONR'₂, R' ayant les significations ci-dessus ;
R² est choisi parmi des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques, aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR'₂, R' ayant les significations ci-dessus ;
R³ est choisi parmi -CN, -SH, -SR", OR", -(C=O)-R", des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques, aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR'₂, R' et R" ayant les significations ci-dessus ;
R⁴ est choisi dans le groupe constitué par -X, -CN, -SH, -SR", OR",-(C=O)-R", des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques, aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR'₂, R' et R" ayant les significations ci-dessus ;
R⁵ correspond à la formule (II-a) 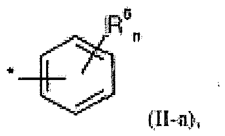 dans laquelle
n est un nombre entier valant 0, 1, 2 ou 3 ;
R⁶ est choisi parmi un atome d'hydrogène, des groupes alkyle en C₁-C₁₂ linéaires, des groupes alkyle en C₃-C₈ ramifiés ou cycliques, des atomes d'halogène, des groupes halogénoalkyle(C₁-C₅)
et leurs sels.

2. Fluoroalkylphénylamidines selon la revendication 1, dans lesquelles
A est O ;
x est un nombre entier, choisi parmi 2 et 3 ;
R¹ est choisi dans le groupe constitué par un atome d'hydrogène, un groupe mercapto (-SH) ou des groupes alkyle en C₁-C₈ ;
R² est choisi parmi des groupes alkyle en C₁-C₈ ;
R³ est choisi parmi des groupes alkyle en C₂-C₈ linéaires, des groupes alkyle en C₃-C₈ ramifiés et alicycliques ;
et
R⁴ est choisi dans le groupe constitué par -X, des groupes alkyle en C₁-C₈ linéaires ou ramifiés et des groupes halogénoalkyle(C₁-C₅).

3. Fluoroalkylphénylamidines selon l'une quelconque des revendications 1 et 2, dans lesquelles
A est O ;
x est un nombre entier, choisi parmi 1, 2 et 3 ;
R¹ est choisi dans le groupe constitué par un atome d'hydrogène, les groupes méthyle et éthyle ;
R² est choisi dans le groupe constitué par les groupes méthyle et éthyle ;
R³ est choisi dans le groupe constitué par les groupes méthyle, éthyle et cyclopropyle ;
R⁴ peut être choisi parmi les atomes de Cl et F et les groupes -CF₃, -CF₂H, -CH₂F et méthyle ;
et R⁵ est choisi dans les groupes (i) à (iii)
(i) groupes de formule (II-g) 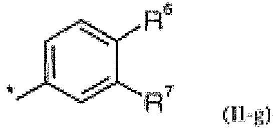 dans laquelle
R⁶ est un atome d'halogène, un groupe alkyle en C₁-C₈, un groupe halogéno-alkyle(C₁-C₅) et
R⁷ est un atome d'hydrogène, d'halogène, chlore ou fluor, un groupe alkyle en C₁-C₈ ou un groupe halogénoalkyle(C₁-C₅),
ou R⁶ et R⁷ peuvent former ensemble avec les atomes du cycle auxquels ils sont liés ou avec d'autres atomes, choisis parmi N, O, P et S, un cycle à quatre à sept chaînons, qui peut être substitué par un ou plusieurs groupes X, R', OR', SR', NR'₂, SiR'₃, R' ayant les significations indiquées plus haut ;
X est un atome d'halogène qui est choisi parmi le fluor, le chlore, le brome et l'iode ;
(ii) groupes de formule (II-h) 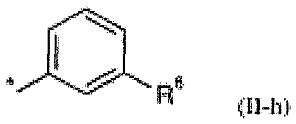 dans laquelle
R⁶ peut être un atome d'halogène, un groupe alkyle en C₁-C₈ linéaire ou ramifié, un groupe cycloalkyle en C₃-C₁₂, aryle en C₅-C₁₈, aralkyle en C₇-C₁₉ ou alkaryle en C₇-C₁₉, les groupes nommés précédemment pouvant être substitués par SiR'₃-, -OR' et -CN, R' ayant les significations indiquées précédemment.

4. Fluoroalkylphénylamidines selon l'une quelconque des revendications 1 à 3, choisies dans le groupe constitué par (1) la N-éthyl-N-méthyl-N'-[4-(3-tert-butyl-4-chlorophénoxy)-5-fluorométhyl-2-méthyl-phényl]formamidine, (2) la N-éthyl-N-méthyl-N'-[4-(4-tert-butyl-phényl)-5-fluorométhyl-2-méthylphényl]-formamidine, (3) la N-éthyl-N-méthyl-N'-[4-(3-tert-butyl-4-chlorophénoxy)-5-trifluorométhyl-2-méthyl-phényl]formamidine, (4) la N-éthyl-N-méthyl-N'-[4-(3-isopropyl-phénoxy)-5-trifluorméthyl-2-méthylphényl]-formamidine, (5) la N-éthyl-N-méthyl-N'-[4-(3-trifluorométhyl-4-chlorophénoxy)-5-trifluorométhyl-2-méthylphényl]formamidine, (6) la N-propyl-N-méthyl-N'-[4-(3-trifluorométhyl-4-chlorophénoxy)-5-trifluoro-méthyl-2-méthylphényl]formamidine, (8) la N,N-diméthyl-N'-[4-(3-tert-butyl-4-chlorophénoxy)-5-trifluorométhyl-2-méthylphényl]-formamidine, (9) la N-éthyl-N-méthyl-N'-[4-(3-tert-butyl-4-chlorophénoxy)-5-difluorométhyl-2-méthylphényl]formamidine (10) la N-éthyl-N-méthyl-N'-[4-(3,4-dichlorophénoxy)-5-difluorométhyl-2-méthyl-phényl]formamidine, (11) la N-éthyl-N-méthyl-N'-[4-(3-tert-butyl-phénoxy)-5-trifluorométhyl-2-méthylphényl]-formamidine, (12) la N-éthyl-N-méthyl-N' -[4-(4-tert-butyl-phényl)-5-trifluorméthyl-2-éthylphényl]-formamidine, (13) la N-éthyl-N-méthyl-N'-[4-phénoxy-5-difluorométhyl-2-méthylphényl]formamidine, (15) la N-éthyl-N-méthyl-N'-{4-[3-(1-hydroxyéthyl)-phénoxy]-5-difluorométhyl-2-méthylphényl}formamidine, (16) la N-éthyl-N-méthyl-N'-{4-[3-(1-fluoréthyl)-phénoxy]-5-difluorométhyl-2-méthylphényl}formamidine, (17) la N-éthyl-N-méthyl-N'-[4-(3-isopropyl-4- chlorophénoxy)-5-difluorométhyl-2-méthylphényl]formamidine, (18) la N-éthyl-N-méthyl-N'-[4-(3-isopropyl-phénoxy)-5-difluorométhyl-2-méthylphényl]formamidine.

5. Procédé pour la préparation des fluoroalkylphénylamidines selon l'une quelconque des revendications 1 à 4, comprenant au moins l'une des étapes (a) à (n) suivantes :
(a) mise en réaction de dérivés de nitrobenzène de formule (III) avec des alcools ou phénols de formule (II) selon le schéma de réaction suivant : 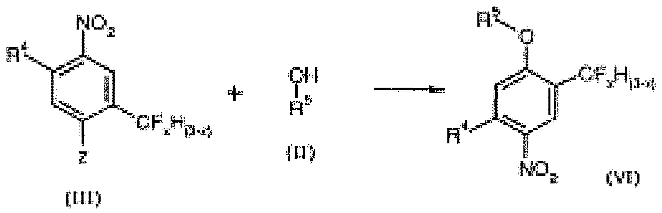
(b) mise en réaction de dérivés de nitrophénol de formule (V) avec des dérivés de phényle de formule (IV) selon le schéma de réaction suivant : 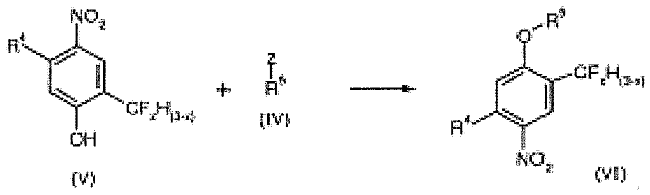
(c) mise en réaction d'anilines de formule (VII) avec des alcools ou phénols de formule (II) selon le schéma de réaction suivant : 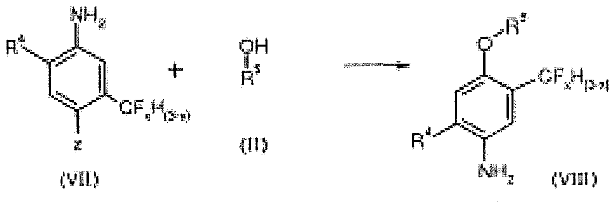
(d) mise en réaction d'aminophénols de formule (XII) avec des dérivés de phényle de formule (IV) selon le schéma de réaction suivant : 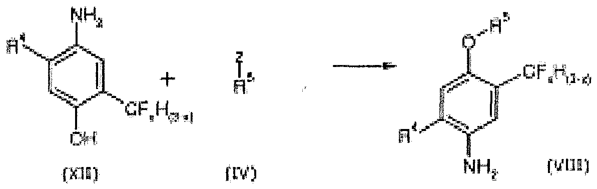
(e) réduction des fluoroalkylnitrophénols de formule (VI) en fluoroalkylanilines de formule (VIII) selon le schéma de réaction suivant : 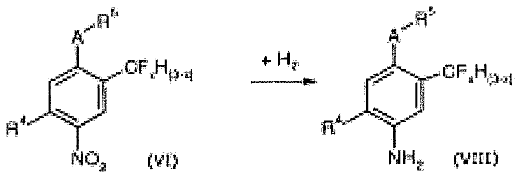
(f) mise en réaction des éthers d'anilines de formule (VIII) avec
(i) des aminoacétals de formule (XIII) ou
(ii) avec des amides de formule (XIV) ou
(iii)avec des amines de formule (XV) en présence d'orthoesters de formule (XVI) selon le schéma de réaction suivant : 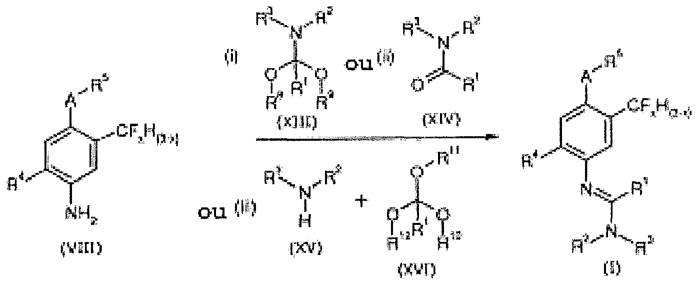
(g) mise en réaction des aminophénols de formule (XII) avec
(i) des aminoacétals de formule (XIII) ou
(ii) avec des amides de formule (XIV) ou
(iii)avec des amines de formule (XV) en présence d'orthoesters de formule (XVI)
selon le schéma de réaction suivant : 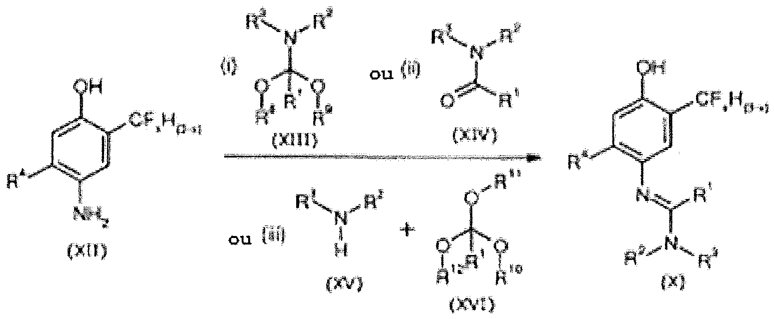
(h) mise en réaction des aminophénols de formule (VII) avec
(i) des aminoacétals de formule (XIII) ou
(ii) avec des amides de formule (XIV) ou
(iii) avec des amines de formule (XV) en présence d'orthoesters de formule (XVI) selon le schéma de réaction suivant : 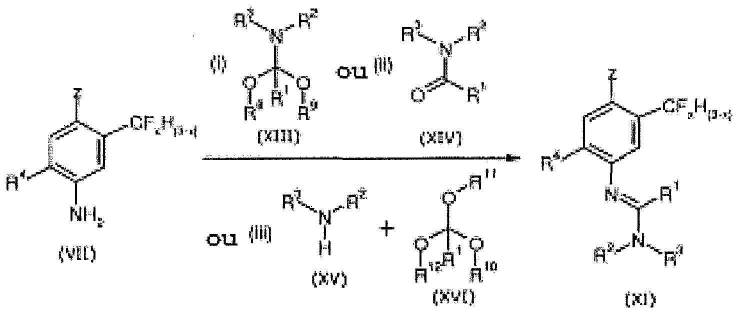
(i) mise en réaction d'amidines de formule (XI) avec des alcools ou phénols de formule (II) selon le schéma de réaction suivant : 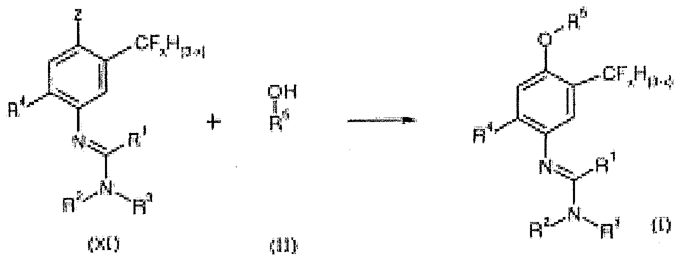
(j) mise en réaction d'amidines de formule (XI) avec des dérivés de phényle de formule (IV) selon le schéma de réaction suivant : 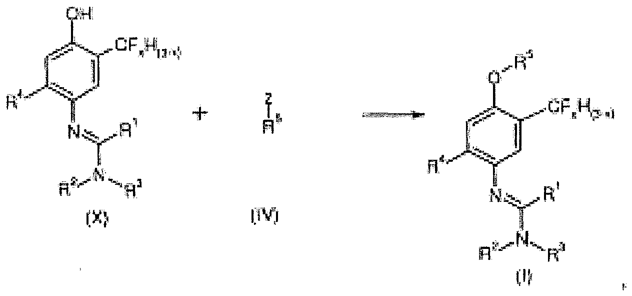
(k) mise en réaction de fluoroalkylanilines de formule (VII) avec des dérivés d'acide boronique de formule (XVII) selon le schéma de réaction suivant : 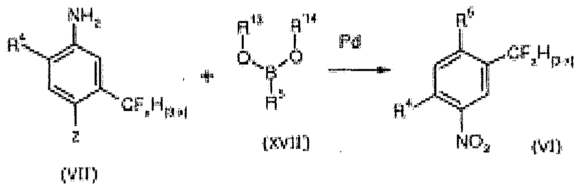 dans les formules décrites précédemment
x, Z, R¹ à R⁵ ayant les significations indiquées plus haut ;
R¹³ et R¹⁴ étant choisis indépendamment l'un de l'autre dans le groupe constitué par un atome d'hydrogène, des groupes alkyle en C₁-C₁₂, alcényle en C₂-C₁₂, alcynyle en C₂-C₁₂ ou aryle en C₅-C₁₈ ou arylalkyle en C₇-C₁₉ et pouvant former, conjointement avec les atomes d'oxygène auxquels ils sont liés, un cycle à cinq, six ou sept chaînons ;
(l) mise en réaction de fluoroalkylanilines de formule (VII) avec des aldéhydes ou cétones de formule (XVIII) selon le schéma de réaction suivant : 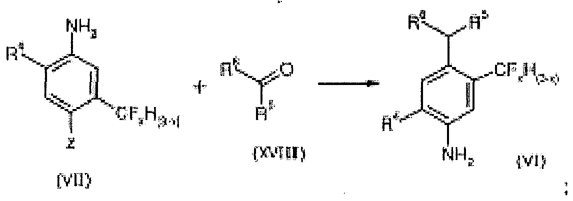
(m) mise en réaction de fluoroalkylnitrobenzènes de formule (III) avec des dérivés d'acide boronique de formule (XVII) selon le schéma de réaction suivant : 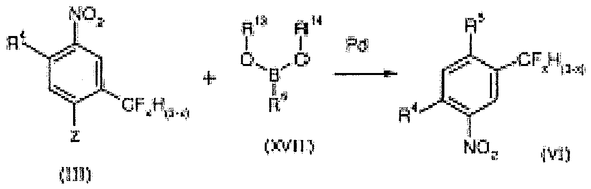
(n) mise en réaction de fluoroalkylnitrobenzènes de formule (III) avec des aldéhydes ou cétones de formule (XVIII) selon le schéma de réaction suivant : 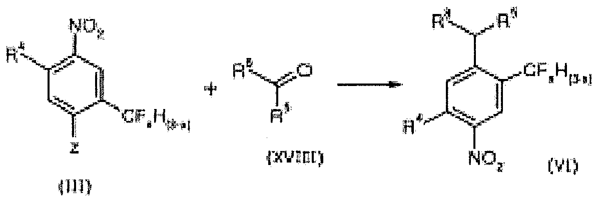 dans les formules décrites précédemment
x, Z, R¹ à R⁸ ayant les significations indiquées plus haut ;
R¹³ et R¹⁴ étant choisis indépendamment l'un de l'autre dans le groupe constitué par un atome d'hydrogène, des groupes alkyle en C₁-C₁₂, alcényle en C₂-C₁₂, alcynyle en C₂-C₁₂ ou aryle en C₅-C₁₈ ou arylalkyle en C₇-C₁₉ et pouvant former, conjointement avec les atomes d'oxygène auxquels ils sont liés, un cycle à cinq, six ou sept chaînons ;

6. Agent destiné à la lutte contre des micro-organismes indésirables, comprenant au moins une fluoroalkylphénylamidine selon l'une quelconque des revendications 1 à 4.

7. Utilisation non thérapeutique d'une fluoroalkylphénylamidine selon l'une quelconque des revendications 1 à 4 ou de mélanges de telles amidines, pour la lutte contre des micro-organismes indésirables.

8. Procédé non thérapeutique pour la lutte contre des micro-organismes indésirables, **caractérisé en ce qu'**on applique sur les micro-organismes et/ou dans leur habitat des fluoroalkylphénylamidines selon l'une quelconque des revendications 1 à 4.

9. Semence, qui est traitée par au moins une fluoroalkylphénylamidine selon l'une quelconque des revendications 1 à 4.

10. Utilisation de fluoroalkylphénylamidines selon l'une quelconque des revendications 1 à 4 pour le traitement de semences.

11. Utilisation de fluoroalkylphénylamidines selon l'une quelconque des revendications 1 à 4 pour le traitement de plantes transgéniques.

12. Utilisation de fluoroalkylphénylamidines selon l'une quelconque des revendications 1 à 4 pour le traitement de semences de plantes transgéniques.

13. Procédé pour la protection de semences contre des micro-organismes indésirables par utilisation d'une semence traitée par au moins une fluoroalkylphényl-amidine selon l'une quelconque des revendications 1 à 4.
